(19) **Europäisches Patentamt European Patent Office Office européen des brevets**

(11) **EP 4 541 821 A1**

(12) **EUROPEAN PATENT APPLICATION**
published in accordance with Art. 153(4) EPC

(43) Date of publication:
**23.04.2025 Bulletin 2025/17**

(21) Application number: **23823222.7**

(22) Date of filing: **15.06.2023**

(51) International Patent Classification (IPC):
*C07K 16/46* (2006.01)   *C07K 16/28* (2006.01)
*C12N 15/13* (2006.01)   *A61K 39/395* (2006.01)
*A61P 35/00* (2006.01)

(52) Cooperative Patent Classification (CPC):
**A61K 39/395; A61K 45/06; A61P 35/00; C07K 16/00; C07K 16/28; C07K 16/30; C07K 16/32; C07K 16/46; C12N 5/163**

(86) International application number:
**PCT/CN2023/100452**

(87) International publication number:
**WO 2023/241656 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.06.2022   CN 202210676083**

(71) Applicant: **Akeso Biopharma, Inc.
Zhongshan, Guangdong 528437 (CN)**

(72) Inventors:
• **XIA, Yu**
**Zhongshan, Guangdong 528437 (CN)**
• **WANG, Zhongmin**
**Zhongshan, Guangdong 528437 (CN)**
• **ZHANG, Peng**
**Zhongshan, Guangdong 528437 (CN)**
• **LI, Baiyong**
**Zhongshan, Guangdong 528437 (CN)**

(74) Representative: **Cabinet Nony
11 rue Saint-Georges
75009 Paris (FR)**

Remarks:
The complete document including Reference Table(s) and the Sequence Listing(s) can be downloaded from the EPO website

(54) **BISPECIFIC ANTIBODY CONTAINING ANTI-CLDN18.2 ANTIBODY, AND PHARMACEUTICAL COMPOSITION AND USE THEREOF**

(57)   Provided are a bispecific antibody containing an anti-CLDN18.2 antibody, and a pharmaceutical composition and the use thereof. The bispecific antibody comprises a fist protein functional region and a second protein functional region, wherein the first protein functional region is an anti-CLDN18.2 antibody or an antigen-binding fragment thereof, and the second protein functional region targets a target different from CLDN18.2 (for example, CD47). The bispecific antibody has a good biological activity and anti-tumor application prospects.

EP 4 541 821 A1

FIG. 6

**Description**

TECHNICAL FIELD

The present invention belongs to the field of biomedicine, and relates to an anti-CLDN18.2 antibody, a bispecific antibody comprising the anti-CLDN18.2 antibody or the antigen-binding fragment thereof, a pharmaceutical composition thereof, and use thereof. Specifically, the bispecific antibody is an anti-CLDN18.2/anti-CD47 bispecific antibody.

BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor treatment response, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

CD47 is also referred to as integrin associated protein (IAP). CD47 is a five-span transmembrane protein with a molecular weight of about 50 kDa and belongs to the immunoglobulin superfamily. Its extracellular N terminus is an IgV domain and is connected to $\alpha v \beta 3$ (CD51/CD61) and $\alpha IIb\beta 3$ (CD41/CD61) integrins. CD47 is involved in a variety of physiological functions, such as cell transfer, T cell and dendritic cell (DC) activation, and axonal development.

CD47 is expressed on all types of cells including red blood cells, and is highly expressed on almost all types of tumor cells. It is also associated with a poor prognosis. CD47 has two ligands, namely signal regulatory protein-$\alpha$ (SIRP$\alpha$) and thrombin-sensitive protein-1 (TSP1). SIRP$\alpha$ is a receptor transmembrane glycoprotein comprising an immunoglobulin domain, belongs to the SIRP family, and is mainly expressed on phagocytes and nerve cells. In the CD47-SIRP$\alpha$ pathway, CD47 protein binds to SIRP$\alpha$ and phosphorylates its immunoreceptor tyrosine-based inhibitory motif (ITIM), and then intracellularly recruits SHP-1 protein to produce a series of cascade reactions to inhibit macrophage phagocytosis (Matozaki T, Murata Y, Okazawa H, et al., Functions and molecular mechanisms of the CD47-SIRP$\alpha$ signaling pathway. Trends in cell biology, 2009, 19(2): 72-80.). Normal red blood cells are not phagocytosed due to the inhibitory signal generated by the binding of CD47 on the surface of the cell membrane to SIPR$\alpha$ of macrophages (Oldenborg P A, Zheleznyak A, Fang Y F, et al., Role of CD47 as a marker of self on red blood cells. Science, 2000, 288(5473): 2051-2054.). TSP1, a homotrimer composed of 3 peptide chains, is involved in cell proliferation, apoptosis, adhesion, migration, angiogenesis, and other processes through interaction with other cell surface receptors, matrix components, and growth factors (Jiang P, Lagenaur CF, Narayanan V. Integrin-associated Protein Is a Ligand for the P84 Neural Adhesion Molecule. Journal of Biological Chemistry 1999, 274: 559-62).

Macrophages are derived from monocytes, which in turn are derived from precursor cells in the bone marrow. Their main functions are to phagocytose cell debris and pathogens and to activate lymphocytes or other immune cells to respond to the pathogens in the form of fixed cells or free cells. Currently, studies suggest that tumor cells have a mechanism of escaping macrophage phagocytosis. During the growth of tumor cells, specific proteins such as calreticulin are formed on the surface, exposing the identity of the tumor cells, such that the tumor cells are phagocytosed by the attracted macrophages. However, tumor cells with highly expressed CD47 are mistakenly recognized as normal cells by macrophages with SIRP$\alpha$ and thus escape macrophage phagocytosis, since the CD47-SIRP$\alpha$ pathway activates the inhibition of the macrophage phagocytosis (CD47 is upregulated on circulating hematopoietic stem cells and leukemia cells to avoid phagocytosis. Jaiswal S, Jamieson C H M, Pang W W, et al., Cell, 2009, 138(3): 271-285).

The CLDN18.2 protein is an integrin membrane protein present in the epithelial and endothelial tight junction. It consists of 261 amino acids and is one of the Claudins (CLDNs) family members, and its N- and C-termini are both located in cells. The entire protein is expressed on the cell membrane. CLDN18.2 has 4 transmembrane domains, 2 extracellular loops, and 1 intracytoplasmic loop and is involved in the formation of a tight junction structure between cells (Gunzel, D.; Yu, A. S. L. Claudins and the Modulation of Tight Junction Permeability [J]. Physiological Reviews. 2013, 93(2), 525-569).

Extracellular loop 2 of the CLDN18.2 protein is a helix-turn-helix structure and forms a tight junction with the extracellular loop of the CLDN18.2 protein of an adjacent cell through hydrophobic bonds between aromatic residues. The extracellular loops of the CLDN18.2 proteins of adjacent cells can maintain the tight junction between the epithelial cell and the endothelial cell through interactions, regulate intercellular osmotic pressure, maintain the polarity of the epithelial cell and the endothelial cell, and participate in cell proliferation, and can participate in a variety of signal transduction pathways through the carboxyl-termini rich in serine, threonine, and tyrosine (Cao Chenxin, Research progress of transmembrane CLDN18.2 in targeted cancer therapy [J]. International Journal of Biologicals, 2020(01): 35-36-37-38-39-40).

The expression of the CLDN18.2 protein is highly restricted in normal healthy tissues and is only found in differentiated epithelial cells of the gastric mucosa, which is beneficial to maintaining the barrier function of the gastric mucosa. However, the CLDN18.2 protein is frequently abnormally changed during the development and progression of malignant tumors. For example, when gastric epithelial tissues are subjected to malignant transformation, the disturbance of cell polarity will result in the exposure of epitopes of the CLDN18.2 protein on the cell surface. At the same time, the CLDN18.2 gene is also abnormally activated, and it is highly selectively and stably expressed in specific tumor tissues, such as gastric cancer, affects the cell-cell and cell-extracellular matrix adhesion, and affects the maintenance of cell barrier function and polarity, thereby causing changes of intercellular cytokine and ion permeability, damaging the barrier function, and causing proliferative transformation of the cells (Hashimoto Itaru, Oshima Takashi, Claudins and Gastric Cancer: An Overview.[J]. Cancers (Basel), 2022, 14: undefined).

Both CD47 and CLDN18.2 are tumor membrane antigens. Antibodies targeting either CD47 or CLDN18.2 have exhibited anti-tumor activity. However, a plurality of blood cells and components including platelets and red blood cells also express CD47, and CD47 antibody drugs, when bound to red blood cells, platelets, etc., may cause damage to the red blood cells and the platelets by ADCC or ADCP or the like, resulting in severe blood toxicity, and side effects such as anemia and/or thrombocytopenia.

**SUMMARY**

Through intensive studies and creative efforts, the inventors have obtained an anti-CLDN18.2 antibody, and based on this, have developed an anti-CLDN18.2/anti-CD47 bispecific antibody. The inventors have surprisingly found that the anti-CLDN18.2 antibody (also referred to as antibody or antibody of the present invention for short) and the anti-CLDN18.2/anti-CD47 bispecific antibody (also referred to as bispecific antibody or bispecific antibody of the present invention for short) of the present invention have excellent affinity and/or specificity, can bind to tumor cells expressing CD47 and/or CLDN18.2 with high specificity without causing agglutination of or damage to red blood cells, exhibit good safety, and have good anti-tumor prospects. The present invention is detailed below.

One aspect of the present invention relates to an anti-CLDN18.2 antibody or an antigen-binding fragment thereof, wherein the anti-CLDN18.2 antibody comprises a heavy chain variable region comprising HCDR1 to HCDR3 and a light chain variable region comprising LCDR1 to LCDR3, wherein:

an amino acid sequence of HCDR1 is set forth in SEQ ID NO: 31, an amino acid sequence of HCDR2 is set forth in SEQ ID NO: 32, and an amino acid sequence of HCDR3 is set forth in SEQ ID NO: 33, and
an amino acid sequence of LCDR1 is set forth in SEQ ID NO: 34, an amino acid sequence of LCDR2 is set forth in SEQ ID NO: 35, and an amino acid sequence of LCDR3 is set forth in SEQ ID NO: 36.

In some embodiments of the present invention, the anti-CLDN18.2 antibody or the antigen-binding fragment thereof is provided, wherein,

an amino acid sequence of the heavy chain variable region of the anti-CLDN18.2 antibody is selected from SEQ ID NO: 28, SEQ ID NO: 37, SEQ ID NO: 39, and SEQ ID NO: 41; and
an amino acid sequence of the light chain variable region of the anti-CLDN18.2 antibody is selected from SEQ ID NO: 30, SEQ ID NO: 43, SEQ ID NO: 45, and SEQ ID NO: 47.

In some embodiments of the present invention, the anti-CLDN18.2 antibody or the antigen-binding fragment thereof is provided, wherein,

the amino acid sequence of the heavy chain variable region is set forth in SEQ ID NO: 28, and the amino acid sequence of the light chain variable region is set forth in SEQ ID NO: 30;
the amino acid sequence of the heavy chain variable region is set forth in SEQ ID NO: 37, and the amino acid sequence of the light chain variable region is set forth in SEQ ID NO: 43;
the amino acid sequence of the heavy chain variable region is set forth in SEQ ID NO: 37, and the amino acid sequence of the light chain variable region is set forth in SEQ ID NO: 45;
the amino acid sequence of the heavy chain variable region is set forth in SEQ ID NO: 37, and the amino acid sequence of the light chain variable region is set forth in SEQ ID NO: 47;
the amino acid sequence of the heavy chain variable region is set forth in SEQ ID NO: 39, and the amino acid sequence of the light chain variable region is set forth in SEQ ID NO: 43;
the amino acid sequence of the heavy chain variable region is set forth in SEQ ID NO: 39, and the amino acid sequence of the light chain variable region is set forth in SEQ ID NO: 45;
the amino acid sequence of the heavy chain variable region is set forth in SEQ ID NO: 39, and the amino acid sequence of the light chain variable region is set forth in SEQ ID NO: 47;
the amino acid sequence of the heavy chain variable region is set forth in SEQ ID NO: 41, and the amino acid sequence of the light chain variable region is set forth in SEQ ID NO: 43;

the amino acid sequence of the heavy chain variable region is set forth in SEQ ID NO: 41, and the amino acid sequence of the light chain variable region is set forth in SEQ ID NO: 45;

or

the amino acid sequence of the heavy chain variable region is set forth in SEQ ID NO: 41, and the amino acid sequence of the light chain variable region is set forth in SEQ ID NO: 47.

In some embodiments of the present invention, the anti-CLDN18.2 antibody or the antigen-binding fragment thereof is provided, wherein a heavy chain constant region of the antibody is Ig gamma-1 chain C region (e.g., having an amino acid sequence set forth in SEQ ID NO: 61) or Ig gamma-4 chain C region (e.g., NCBI ACCESSION: P01861.1); a light chain constant region of the antibody is Ig kappa chain C region (e.g., NCBI ACCESSION: P01834).

In some embodiments of the present invention, the anti-CLDN18.2 antibody or the antigen-binding fragment thereof is provided, wherein the anti-CLDN18.2 antibody or the antigen-binding fragment thereof is selected from Fab, Fab', F(ab')$_2$, Fd, Fv, dAb, a complementarity determining region fragment, a single chain fragment variable, a humanized antibody, a chimeric antibody, or a diabody.

In some embodiments of the present invention, the anti-CLDN18.2 antibody or the antigen-binding fragment thereof is provided, wherein,

the antibody comprises a non-CDR region derived from a non-murine species, such as from a human antibody.

In some embodiments of the present invention, the anti-CLDN18.2 antibody or the antigen-binding fragment thereof is provided, wherein an $EC_{50}$ of the anti-CLDN18.2 antibody for binding to a cell expressing CLDN18.2 is less than or equal to 15 nM, less than or equal to 10 nM, less than or equal to 9 nM, less than or equal to 8 nM, less than or equal to 7 nM, less than or equal to 6 nM, or less than or equal to 5 nM; preferably, the $EC_{50}$ is determined by FACS (flow cytometry). In one embodiment of the present invention, the cell expressing CLDN18.2 is a CHO-K1 cell expressing CLDN18.2. In one embodiment of the present invention, the cell expressing CLDN18.2 is a CHO-K1 cell overexpressing CLDN18.2.

In some embodiments of the present invention, the anti-CLDN18.2 antibody or the antigen-binding fragment thereof is provided, wherein an $EC_{50}$ of the anti-CLDN18.2 antibody for binding to a cell expressing both CLDN18.2 and CD47 is less than or equal to 10 nM, less than or equal to 5 nM, or less than or equal to 2 nM; preferably, the $EC_{50}$ is determined by FACS. In one embodiment of the present invention, the cell expressing both CLDN18.2 and CD47 is a CHO-K1 cell expressing both CLDN18.2 and CD47. In one embodiment of the present invention, the cell expressing both CLDN18.2 and CD47 is a CHO-K1 cell overexpressing both CLDN18.2 and CD47.

In some embodiments of the present invention, the anti-CLDN18.2 antibody is an anti-CLDN18.2 monoclonal antibody.

The present invention also relates to an anti-CLDN18.2 antibody or an antigen-binding fragment thereof, wherein the anti-CLDN18.2 antibody is a monoclonal antibody produced by a hybridoma cell line LT020 deposited at China Center for Type Culture Collection (CCTCC) with a CCTCC designation of CCTCC NO. C2022124.

The present invention also relates a hybridoma cell line LT020 deposited at China Center for Type Culture Collection (CCTCC) with a CCTCC designation of CCTCC NO. C2022124.

Another aspect of the present invention relates to an isolated nucleic acid molecule encoding the anti-CLDN18.2 antibody or the antigen-binding fragment thereof according to any aspect of the present invention.

Yet another aspect of the present invention relates to a recombinant vector comprising the isolated nucleic acid molecule of the present invention.

Yet another aspect of the present invention relates to a host cell comprising the isolated nucleic acid molecule of the present invention or the recombinant vector of the present invention.

Yet another aspect of the present invention relates to an antibody-drug conjugate comprising an antibody or an antigen-binding fragment thereof and a small molecule drug, wherein the antibody or the antigen-binding fragment thereof is the anti-CLDN18.2 antibody or the antigen-binding fragment thereof according to any aspect of the present invention; preferably, the small molecule drug is a small molecule cytotoxic drug; more preferably, the small molecule drug is an anti-tumor chemotherapeutic drug.

The chemotherapeutic drug may be a conventional anti-tumor chemotherapeutic drug, such as an alkylating agent, an antimetabolite, an anti-tumor antibiotic, a plant-based anticancer agent, a hormone, and an immunological agent.

In one or more embodiments of the present invention, the antibody-drug conjugate is provided, wherein the antibody or the antigen-binding fragment thereof is linked to the small molecule drug via a linker; the linker may be one known to those skilled in the art; for example, the linker is a hydrazone bond, a disulfide bond, or a peptide bond.

In one or more embodiments of the present invention, the antibody-drug conjugate is provided, wherein a molar ratio of the antibody or the antigen-binding fragment thereof to the small molecule drug is 1:(2-4), e.g., 1:2, 1:3, or 1:4.

Yet another aspect of the present invention relates to a pharmaceutical composition comprising an effective amount of the anti-CLDN 18.2 antibody or the antigen-binding fragment thereof according to any aspect of the present invention or the antibody-drug conjugate according to any aspect of the present invention, wherein optionally, the pharmaceutical composition further comprises one or more pharmaceutically acceptable auxiliary materials.

In some embodiments of the present invention, the pharmaceutical composition further comprises an effective amount of an anti-CD47 antibody or an antigen-binding fragment thereof, wherein preferably, the anti-CD47 antibody comprises a heavy chain variable region comprising HCDR1 to HCDR3 and a light chain variable region comprising LCDR1 to LCDR3, wherein:

an amino acid sequence of HCDR1 is set forth in SEQ ID NO: 5, an amino acid sequence of HCDR2 is set forth in SEQ ID NO: 6, and an amino acid sequence of HCDR3 is set forth in SEQ ID NO: 7, and

an amino acid sequence of LCDR1 is set forth in SEQ ID NO: 8, an amino acid sequence of LCDR2 is set forth in SEQ ID NO: 9, and an amino acid sequence of LCDR3 is set forth in SEQ ID NO: 10.

In some embodiments of the present invention, the pharmaceutical composition is provided, wherein

an amino acid sequence of the heavy chain variable region of the anti-CD47 antibody is selected from SEQ ID NO: 2, SEQ ID NO: 12, SEQ ID NO: 16, SEQ ID NO: 20, and SEQ ID NO: 24; and

an amino acid sequence of the light chain variable region of the anti-CD47 antibody is selected from SEQ ID NO: 4, SEQ ID NO: 14, SEQ ID NO: 18, SEQ ID NO: 22, and SEQ ID NO: 26;

wherein preferably, in the anti-CD47 antibody:

the amino acid sequence of the heavy chain variable region is set forth in SEQ ID NO: 2, and the amino acid sequence of the light chain variable region is set forth in SEQ ID NO: 4;

the amino acid sequence of the heavy chain variable region is set forth in SEQ ID NO: 12, and the amino acid sequence of the light chain variable region is set forth in SEQ ID NO: 14;

the amino acid sequence of the heavy chain variable region is set forth in SEQ ID NO: 12, and the amino acid sequence of the light chain variable region is set forth in SEQ ID NO: 18;

the amino acid sequence of the heavy chain variable region is set forth in SEQ ID NO: 12, and the amino acid sequence of the light chain variable region is set forth in SEQ ID NO: 22;

the amino acid sequence of the heavy chain variable region is set forth in SEQ ID NO: 12, and the amino acid sequence of the light chain variable region is set forth in SEQ ID NO: 26;

the amino acid sequence of the heavy chain variable region is set forth in SEQ ID NO: 16, and the amino acid sequence of the light chain variable region is set forth in SEQ ID NO: 14;

the amino acid sequence of the heavy chain variable region is set forth in SEQ ID NO: 16, and the amino acid sequence of the light chain variable region is set forth in SEQ ID NO: 18;

the amino acid sequence of the heavy chain variable region is set forth in SEQ ID NO: 16, and the amino acid sequence of the light chain variable region is set forth in SEQ ID NO: 22;

the amino acid sequence of the heavy chain variable region is set forth in SEQ ID NO: 16, and the amino acid sequence of the light chain variable region is set forth in SEQ ID NO: 26;

the amino acid sequence of the heavy chain variable region is set forth in SEQ ID NO: 20, and the amino acid sequence of the light chain variable region is set forth in SEQ ID NO: 14;

the amino acid sequence of the heavy chain variable region is set forth in SEQ ID NO: 20, and the amino acid sequence of the light chain variable region is set forth in SEQ ID NO: 18;

the amino acid sequence of the heavy chain variable region is set forth in SEQ ID NO: 20, and the amino acid sequence of the light chain variable region is set forth in SEQ ID NO: 22;

the amino acid sequence of the heavy chain variable region is set forth in SEQ ID NO: 20, and the amino acid sequence of the light chain variable region is set forth in SEQ ID NO: 26;

the amino acid sequence of the heavy chain variable region is set forth in SEQ ID NO: 24, and the amino acid sequence of the light chain variable region is set forth in SEQ ID NO: 14;

the amino acid sequence of the heavy chain variable region is set forth in SEQ ID NO: 24, and the amino acid sequence of the light chain variable region is set forth in SEQ ID NO: 18;

the amino acid sequence of the heavy chain variable region is set forth in SEQ ID NO: 24, and the amino acid sequence of the light chain variable region is set forth in SEQ ID NO: 22;

or

the amino acid sequence of the heavy chain variable region is set forth in SEQ ID NO: 24, and the amino acid sequence of the light chain variable region is set forth in SEQ ID NO: 26.

In some embodiments of the present invention, the pharmaceutical composition is provided, wherein a heavy chain constant region of the anti-CD47 antibody is Ig gamma-1 chain C region (e.g., NCBI ACCESSION: P01857) or Ig gamma-4 chain C region (e.g., NCBI ACCESSION: P01861.1); a light chain constant region of the anti-CD47 antibody is Ig kappa chain C region (e.g., NCBI ACCESSION: P01834).

Yet another aspect of the present invention relates to a bispecific antibody, comprising:

a first protein functional region targeting CLDN18.2, and
a second protein functional region targeting a target other than CLDN18.2 (e.g., CD47),
wherein:
the first protein functional region is the anti-CLDN18.2 antibody or the antigen-binding fragment thereof according to any aspect of the present invention.

Unless otherwise specified, the bispecific antibody of the present invention is an anti-CLDN18.2/anti-CD47 bispecific antibody.

In some embodiments of the present invention, the bispecific antibody is provided, wherein the second protein functional region is an anti-CD47 antibody or an antigen-binding fragment thereof, wherein:
the anti-CD47 antibody comprises a heavy chain variable region comprising HCDR1 to HCDR3 and a light chain variable region comprising LCDR1 to LCDR3, wherein:

an amino acid sequence of HCDR1 is set forth in SEQ ID NO: 5, an amino acid sequence of HCDR2 is set forth in SEQ ID NO: 6, and an amino acid sequence of HCDR3 is set forth in SEQ ID NO: 7, and
an amino acid sequence of LCDR1 is set forth in SEQ ID NO: 8, an amino acid sequence of LCDR2 is set forth in SEQ ID NO: 9, and an amino acid sequence of LCDR3 is set forth in SEQ ID NO: 10.

In some embodiments of the present invention, the bispecific antibody is provided, wherein

an amino acid sequence of the heavy chain variable region of the anti-CD47 antibody is selected from SEQ ID NO: 2, SEQ ID NO: 12, SEQ ID NO: 16, SEQ ID NO: 20, and SEQ ID NO: 24; and
an amino acid sequence of the light chain variable region of the anti-CD47 antibody is selected from SEQ ID NO: 4, SEQ ID NO: 14, SEQ ID NO: 18, SEQ ID NO: 22, and SEQ ID NO: 26.

In some embodiments of the present invention, the bispecific antibody is provided, wherein in the anti-CD47 antibody:

the amino acid sequence of the heavy chain variable region is set forth in SEQ ID NO: 2, and the amino acid sequence of the light chain variable region is set forth in SEQ ID NO: 4;
the amino acid sequence of the heavy chain variable region is set forth in SEQ ID NO: 12, and the amino acid sequence of the light chain variable region is set forth in SEQ ID NO: 14;
the amino acid sequence of the heavy chain variable region is set forth in SEQ ID NO: 12, and the amino acid sequence of the light chain variable region is set forth in SEQ ID NO: 18;
the amino acid sequence of the heavy chain variable region is set forth in SEQ ID NO: 12, and the amino acid sequence of the light chain variable region is set forth in SEQ ID NO: 22;
the amino acid sequence of the heavy chain variable region is set forth in SEQ ID NO: 12, and the amino acid sequence of the light chain variable region is set forth in SEQ ID NO: 26;
the amino acid sequence of the heavy chain variable region is set forth in SEQ ID NO: 16, and the amino acid sequence of the light chain variable region is set forth in SEQ ID NO: 14;
the amino acid sequence of the heavy chain variable region is set forth in SEQ ID NO: 16, and the amino acid sequence of the light chain variable region is set forth in SEQ ID NO: 18;
the amino acid sequence of the heavy chain variable region is set forth in SEQ ID NO: 16, and the amino acid sequence of the light chain variable region is set forth in SEQ ID NO: 22;
the amino acid sequence of the heavy chain variable region is set forth in SEQ ID NO: 16, and the amino acid sequence of the light chain variable region is set forth in SEQ ID NO: 26;
the amino acid sequence of the heavy chain variable region is set forth in SEQ ID NO: 20, and the amino acid sequence of the light chain variable region is set forth in SEQ ID NO: 14;
the amino acid sequence of the heavy chain variable region is set forth in SEQ ID NO: 20, and the amino acid sequence of the light chain variable region is set forth in SEQ ID NO: 18;
the amino acid sequence of the heavy chain variable region is set forth in SEQ ID NO: 20, and the amino acid sequence of the light chain variable region is set forth in SEQ ID NO: 22;
the amino acid sequence of the heavy chain variable region is set forth in SEQ ID NO: 20, and the amino acid sequence of the light chain variable region is set forth in SEQ ID NO: 26;
the amino acid sequence of the heavy chain variable region is set forth in SEQ ID NO: 24, and the amino acid sequence of the light chain variable region is set forth in SEQ ID NO: 14;

the amino acid sequence of the heavy chain variable region is set forth in SEQ ID NO: 24, and the amino acid sequence of the light chain variable region is set forth in SEQ ID NO: 18;

the amino acid sequence of the heavy chain variable region is set forth in SEQ ID NO: 24, and the amino acid sequence of the light chain variable region is set forth in SEQ ID NO: 22;

or

the amino acid sequence of the heavy chain variable region is set forth in SEQ ID NO: 24, and the amino acid sequence of the light chain variable region is set forth in SEQ ID NO: 26.

In some embodiments of the present invention, the pharmaceutical composition product is provided, wherein a heavy chain constant region of the anti-CD47 antibody is Ig gamma-1 chain C region (e.g., NCBI ACCESSION: P01857) or Ig gamma-4 chain C region (e.g., NCBI ACCESSION: P01861.1); a light chain constant region of the anti-CD47 antibody is Ig kappa chain C region (e.g., NCBI ACCESSION: P01834).

In some embodiments of the present invention, the bispecific antibody is provided, wherein the first protein functional region and the second protein functional region are independently a fusion protein of a single chain fragment variable or a half-molecular monovalent antibody (IgG half molecule, IgG-HM).

In some embodiments of the present invention, the bispecific antibody is provided, wherein

the first protein functional region is a fusion protein of a single chain fragment variable, and the second protein functional region is a half-molecular monovalent antibody (IgG half molecule, IgG-HM);

or

the first protein functional region is a half-molecular monovalent antibody (IgG half molecule, IgG-HM), and the second protein functional region is a fusion protein of a single chain fragment variable.

In some embodiments of the present invention, the bispecific antibody is provided, wherein

the first protein functional region is a fusion protein of a single chain fragment variable targeting CLDN18.2, and the second protein functional region is a half-molecular monovalent antibody (IgG half molecule, IgG-HM) targeting CD47;

or

the first protein functional region is a half-molecular monovalent antibody (IgG half molecule, IgG-HM) targeting CLDN18.2, and the second protein functional region is a fusion protein of a single chain fragment variable targeting CD47.

In some embodiments of the present invention, the bispecific antibody is provided, wherein the fusion protein of the single chain fragment variable consists of a single chain fragment variable, a hinge region, and an Fc fragment, or consists of a single chain fragment variable and a heavy chain constant region,

wherein preferably, the hinge region and the Fc fragment are a hinge region and an Fc fragment of human IgG1; preferably, the hinge region and the Fc fragment have an amino acid sequence set forth in SEQ ID NO: 62; the heavy chain constant region is a heavy chain constant region of human IgG1; preferably, the heavy chain constant region has an amino acid sequence set forth in SEQ ID NO: 63.

In some embodiments of the present invention, the bispecific antibody is provided, wherein

the Fc fragment in the fusion protein or the heavy chain constant region of the single chain fragment variable has a Knob mutation (e.g., S354C and T366W mutations); and

a heavy chain constant region of the half-molecular monovalent antibody is a heavy chain constant region of human IgG1, and has a Hole mutation (e.g., Y349C, T366S, L368A, and Y407V mutations).

In some embodiments of the present invention, the bispecific antibody consists of a peptide chain set forth in SEQ ID NO: 53, a peptide chain set forth in SEQ ID NO: 56, and a peptide chain set forth in SEQ ID NO: 59,

wherein preferably, the peptide chain set forth in SEQ ID NO: 53 and the peptide chain set forth in SEQ ID NO: 56 are linked by one or more disulfide bonds of a hinge region, and the peptide chain set forth in SEQ ID NO: 56 and the peptide chain set forth in SEQ ID NO: 59 are linked by one or more disulfide bonds;

preferably, the peptide chain set forth in SEQ ID NO: 53 and the peptide chain set forth in SEQ ID NO: 56 are linked by two disulfide bonds of a hinge region, and the peptide chain set forth in SEQ ID NO: 56 and the peptide chain set forth in SEQ ID NO: 59 are linked by one disulfide bond.

In some embodiments of the present invention, the bispecific antibody is provided, wherein

an $EC_{50}$ of the bispecific antibody for binding to a cell expressing CLDN18.2 is less than or equal to 20 nM, less than or equal to 15 nM, or less than or equal to 12 nM; preferably, the $EC_{50}$ is determined by FACS;
and/or
an $EC_{50}$ of the bispecific antibody for binding to CD47 on red blood cell membrane surface is greater than or equal to 20 nM, greater than or equal to 40 nM, or greater than or equal to 50 nM;
preferably, the $EC_{50}$ is determined by FACS. In one embodiment of the present invention, the cell expressing CLDN18.2 is a CHO-K1 cell expressing CLDN18.2. In one embodiment of the present invention, the cell expressing CLDN18.2 is a CHO-K1 cell overexpressing CLDN18.2.

In some embodiments of the present invention, the bispecific antibody is provided, wherein an $EC_{50}$ of the bispecific antibody for binding to a cell expressing both CLDN18.2 and CD47 is less than or equal to 10 nM, less than or equal to 5 nM, or less than or equal to 2 nM; preferably, the $EC_{50}$ is determined by FACS. In one embodiment of the present invention, the cell expressing both CLDN18.2 and CD47 is a CHO-K1 cell expressing both CLDN18.2 and CD47. In one embodiment of the present invention, the cell expressing both CLDN18.2 and CD47 is a CHO-K1 cell overexpressing both CLDN18.2 and CD47.

In some embodiments of the present invention, the bispecific antibody does not induce agglutination of red blood cells at a concentration of 3000 nM or less.

In some embodiments of the present invention, the bispecific antibody has ADCP activity, ADCC activity, and CDC activity.

In some embodiments of the present invention, the anti-CD47 antibody is an anti-CD47 monoclonal antibody.

Yet another aspect of the present invention relates to an isolated nucleic acid molecule encoding the bispecific antibody according to any aspect of the present invention.

Yet another aspect of the present invention relates to a vector comprising the isolated nucleic acid molecule of the present invention.

Yet another aspect of the present invention relates to a host cell comprising the isolated nucleic acid molecule of the present invention or the vector of the present invention.

Yet another aspect of the present invention relates to a pharmaceutical composition comprising an effective amount of the bispecific antibody according to any aspect of the present invention, and one or more pharmaceutically acceptable auxiliary materials.

Yet another aspect of the present invention relates to use of the anti-CLDN18.2 antibody or the antigen-binding fragment thereof according to any aspect of the present invention or the bispecific antibody according to any aspect of the present invention in preparing a medicament for treating or preventing a tumor,

wherein preferably, the tumor is a CD47 and/or CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

The anti-CLDN18.2 antibody or the antigen-binding fragment thereof according to any aspect of the present invention or the bispecific antibody according to any aspect of the present invention is used in treating or preventing a tumor,

wherein preferably, the tumor is a CD47 and/or CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

Yet another aspect of the present invention relates to a method for treating or preventing a tumor, comprising a step of administering to a subject in need an effective amount of the anti-CLDN18.2 antibody or the antigen-binding fragment thereof according to any aspect of the present invention or the bispecific antibody according to any aspect of the present invention,

wherein preferably, the tumor is a CD47 and/or CLDN18.2-positive tumor;

preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

In some embodiments of the present invention, the method for treating or preventing a tumor is provided, wherein drug administration is performed before or after surgery and/or before or after radiotherapy.

In some embodiments of the present invention, the method for treating or preventing a tumor is provided, wherein

the anti-CLDN18.2 antibody or the antigen-binding fragment thereof or the bispecific antibody is administered at a unit dose of 0.1-100 mg per kg body weight, preferably 5-50 mg or 5-15 mg per kg body weight,

preferably, drug administration is performed once every 3 days, every 4 days, every 5 days, every 6 days, every 10 days, every 1 week, every 2 weeks, or every 3 weeks;

preferably, a route of administration is intravenous drip infusion or intravenous injection.

In the present invention, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present invention, the definitions and explanations of the relevant terms are provided below.

As used herein, the term $EC_{50}$ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into μ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) is replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402. In some cases, the antigen-binding fragment of the antibody is a diabody, in which the $V_H$ and $V_L$ domains are expressed on a single polypeptide chain. However, the linker used is too short to allow the pairing of the two domains on the same chain. Thus the domains are forced to pair with the complementary domains on the other chain and two antigen-binding sites are generated (see, e.g., Holliger P. et al., Proc. Natl. Acad. Sci. USA, 1993; 90: 6444-6448 and Poljak R.J. et al., Structure, 1994; 2: 1121-1123).

As used herein, the term "single chain fragment variable (ScFv)" refers to a molecule comprising an antibody heavy chain variable region ($V_H$) and an antibody light chain variable region ($V_L$) linked via a linker. The $V_L$ and $V_H$ domains are paired to form a monovalent molecule by a linker that enables them to produce a single polypeptide chain (see, e.g., Bird et al., Science, 1988; 242:423-426 and Huston et al., Proc. Natl. Acad. Sci. USA, 1988; 85:5879-5883). Such scFv molecules may have the following general structures: NH2-$V_L$-linker fragment-$V_H$-COOH or NH2-$V_H$-linker fragment-$V_L$-COOH. An appropriate linker in the prior art consists of GGGGS amino acid sequence repeats or a variant thereof. For example, a linker having the amino acid sequence (GGGGS)4 may be used, but variants thereof may also be used (Holliger et al., Proc. Natl. Acad. Sci. USA, 1993; 90: 6444-6448). Other linkers that can be used in the present invention are described in Alfthan et al., Protein Eng., 1995; 8: 725-731, Choi et al., Eur. J. Immunol., 2001; 31:94-106, Hu et al., Cancer Res., 1996; 56: 3055-3061, Kipriyanov et al., J. Mol. Biol., 1999; 293: 41-56 and Roovers et al., Cancer Immunology, Immunotherapy, 2001, 50(1): 51-59.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as λ phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis, fungal cells such as yeast cells or aspergillus, insect cells such as S2 drosophila cells or Sf9, or animal cells such as fibroblasts, CHO cells, GS cells, COS cells, NSO cells, HeLa* cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity ($K_D$) of less than about $10^{-5}$ M, e.g., less than about $10^{-6}$ M, $10^{-7}$ M, $10^{-8}$ M, $10^{-9}$ M, or $10^{-10}$ M or less.

As used herein, the term "$K_D$" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., CLDN18.2 protein) with a dissociation equilibrium constant ($K_D$) of less than about $10^{-5}$ M, such as less than about $10^{-6}$ M, $10^{-7}$ M, $10^{-8}$ M, $10^{-9}$ M, or $10^{-10}$ M or less. $K_D$ can be determined using methods known to those skilled in the art, e.g., using a Fortebio molecular interaction instrument.

As used herein, the terms "monoclonal antibody" and "mAb" have the same meaning and are used interchangeably; the terms "polyclonal antibody" and "pAb" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or an excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants, and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

As used herein, when referring to the amino acid sequence of a CD47 protein (NCBI GenBank: NP_001768.1), it includes the full length of the CD47 protein, or the extracellular fragment CD47 ECD of CD47, or a fragment comprising CD47 ECD, and it also includes a fusion protein of the full length of the CD47 protein or a fusion protein of CD47 ECD, such as a fragment fused to an Fc protein fragment of mouse or human IgG (mFc or hFc). However, those skilled in the art will appreciate that in the amino acid sequence of the CD47 protein, mutations or variations (including but not limited to, substitutions, deletions, and/or additions) can be naturally produced or artificially introduced without affecting biological functions thereof. Therefore, in the present invention, the term "CD47 protein" shall include all such sequences, including their natural or artificial variants. In addition, when the sequence fragment of the CD47 protein is described, the CD47 protein also includes the corresponding sequence fragments in natural or artificial variants thereof.

As used herein, when referring to the amino acid sequence of the CLDN18.2 protein (NCBI GenBank: NP_001002026.1), it includes the full length of the CLDN18.2 protein, or the extracellular fragment CLDN18.2 ECD of CLDN18.2, or a fragment comprising CLDN18.2 ECD, and it also includes a fusion protein of the full length of the CLDN18.2 protein or a fusion protein of CLDN18.2 ECD, such as a fragment fused to an Fc protein fragment of mouse or human IgG (mFc or hFc). However, those skilled in the art will appreciate that in the amino acid sequence of the CLDN18.2 protein, mutations or variations (including, but not limited to, substitutions, deletions, and/or additions) can be naturally produced or artificially introduced without affecting biological functions thereof. Therefore, in the present invention, the term "CLDN18.2 protein" shall include all such sequences, including their natural or artificial variants. In addition, when describing a sequence fragment of the CLDN18.2 protein, it also includes the corresponding sequence fragments in their natural or artificial variants.

In the present invention, the term "ADCP" refers to antibody-dependent cellular phagocytosis. The Fc fragment of an antibody that binds to a cell surface antigen binds to the Fc receptor of a phagocytically active cell, such as a macrophage, which in turn mediates phagocytosis of the antibody-bound cells by phagocytic cells.

In the present invention, the term "ADCC" refers to antibody-dependent cell-mediated cytotoxicity. The Fab fragment of an antibody binds to an epitope of a virus-infected cell or a tumor cell, and the Fc fragment of the antibody binds to an Fc receptor (FcR) on the surface of a killer cell (NK cell, macrophage, etc.) to mediate direct killing of the target cells by killer cells.

In the present invention, the term "CDC" refers to complement-dependent cytotoxicity. When an antibody specifically binds to a corresponding antigen on a cell membrane surface, a complex is formed and the complement system is activated, and then an MAC is formed on the surface of a target cell, resulting in subsequent target cell lysis. Complements may cause cell lysis of various bacteria and other pathogenic organisms, and are an important defense mechanism against pathogenic organism infections.

In the present invention, the terms "first" (e.g., first protein functional region or first pharmaceutical product) and "second" (e.g., second protein functional region or second pharmaceutical product) are used for distinguishing or clarity in expression and do not carry typical sequential meanings, unless otherwise specified.

In the present invention, the term "half-molecular monovalent antibody (IgG half molecule, IgG-HM)" is an antibody molecule consisting of 1 heavy chain and 1 light chain of an IgG antibody (e.g., IgG1, IgG2, IgG3, or IgG4), which is a monovalent antibody (see, e.g., FENG Yi-fan et al., Construction and activity analysis of HIV specific monovalent mAb 2G12, Chinese Journal of Viral Diseases, May 2015, 5(3): 171-175).

Beneficial Effects of the Present Invention

The present invention achieves one or more of the following effects:

    (1) the anti-CLDN18.2 antibody of the present invention has excellent affinity and specificity;
    (2) the bispecific antibody of the present invention, for example, can specifically bind to CLDN18.2 well, exerting an antibody-mediated cell killing effect against cells expressing CLDN18.2;

(3) the bispecific antibody of the present invention, for example, can specifically bind to cells expressing both CD47 and CLDN18.2 well, exerting an antibody-mediated cell killing effect against cells expressing both CD47 and CLDN18.2;

(3) the bispecific antibody of the present invention can specifically bind to CD47 and effectively block the binding of CD47 to SIRPα, thereby specifically relieving CD47/SIRPα-mediated immunosuppression and activating immune response;

(4) the first protein functional region and the second protein functional region in the bispecific antibody of the present invention have a synergistic effect;

(5) the bispecific antibody of the present invention does not induce agglutination of red blood cells, has high safety, and can have ADCP, ADCC effect, and CDC effect.

BRIEF DESCRIPTION OF THE DRAWINGS

FIG. 1: FACS assay of binding activity of 8C5.1H3L3, 6F7H1L1(hG4), and AsAb-8C4703 to CHO-K1-CLDN18.2-CD47 cells.

FIG. 2: FACS assay of binding activity of 8C5.1H3L3 and AsAb-8C4703 to CHO-K1-CLDN18.2 cells.

FIG. 3: FACS assay of binding activity of AsAb-8C4703 to red blood cells.

FIG. 4: FACS assay of 6F7H1L1(hG4) in competing with AsAb-8C4703 for binding to CHO-K1-CLDN18.2-CD47 surface antigens.

FIG. 5: Agglutination of normal human red blood cells induced by 6F7H1L1(hG4) and AsAb-8C4703.

FIG. 6: 8C5.1H3L3, 6F7H1L1(hG4), and AsAb-8C4703-mediated phagocytosis of KATOIII-CLDN18.2 cells by HPMM.

FIG. 7: ADCC activity assay of 8C5.1H3L3, AsAb-8C4703, and 6F7H1L1(hG4) against CHO-K1-CLDN18.2-CD47 cells.

FIG. 8: CDC activity assay of 8C5.1H3L3, AsAb-8C4703, and 6F7H1L1(hG4) against CHO-K1-CLDN18.2-CD47 cells.

FIG. 9: 8C5.1H3L3 and IMAB362-mediated phagocytosis of CHO-K1-CLDN18.2 cells by HPMM.

FIG. 10: Binding activity assay of AsAb-8C4703, 8C5.1H3L3, and IMAB362 to antigen human CLDN18.2 by indirect ELISA.

FIG. 11: Binding activity assay of AsAb-8C4703 and 6F7H1L1(hG4) to antigen CD47-Igv-TEV-his by indirect ELISA.

FIG. 12: Detection results of kinetic parameters of binding of 8C5.1H3L3 to CLDN18.2 His.

FIG. 13: Detection results of kinetic parameters of binding of AsAb-8C4703 to CLDN18.2 His.

FIG. 14: Detection results of kinetic parameters of binding of 6F7H1L1(hG4) to CD47 ECD-His.

FIG. 15: Detection results of kinetic parameters of binding of AsAb-8C4703 to CD47 ECD-His.

FIG. 16: Binding activity assay of AsAb-8C4703 and Hu5F9-G4 to T cells.

FIG. 17: Binding activity assay of AsAb-8C4703 and Hu5F9-G4 to B cells.

FIG. 18: Efficacy results of AsAb-8C4703, 6F7H1L1(hG4) single drug, and 8C5.1H3L3 single drug in MC38-CD47-CLDN18.2 xenograft tumor C57BL/6 mouse model.

FIG. 19: Efficacy results of 6F7H1L1(hG4) in combination with 8C5.1H3L3 in MC-38-CD47-CLDN18.2 xenograft tumor C57BL/6 mouse model.

FIG. 20: Effects of AsAb-8C4703, 6F7H1L1(hG4) single drug, and 8C5.1H3L3 single drug on body weight in MC38-CD47-CLDN18.2 xenograft tumor C57BL/6 mouse model.

FIG. 21: Effects of 6F7H1L1(hG4) in combination with 8C5.1H3L3 on body weight in MC-38-CD47-CLDN18.2 xenograft tumor C57BL/6 mouse model.

Hybridoma cell line LT012 was deposited at China Center for Type Culture Collection (CCTCC) on June 21, 2018 with a CCTCC designation of CCTCC NO. C2018135, the depository address being Wuhan University, Wuhan, China, postal code: 430072.

Hybridoma cell line LT020 was deposited at China Center for Type Culture Collection (CCTCC) on May 19, 2022 with a CCTCC designation of CCTCC NO. C2022124, the depository address being Wuhan University, Wuhan, China, postal code: 430072.

Some sequences involved in the present invention are as follows:

6F7 heavy chain variable region:

CAGGTGCAGCTGCAGCAGCCAGGAGCAGAGCTGGTGAGGCCAGGAGCATCCGTGAA

GCTGTCTTGTAAGGCCAGCGGCTACACCTTCACATCCTATTGGATGAACTGGGTGAA

GCAGAGGCCTGGACAGGGACTGGAGTGGATCGGCATGATCGACCCAAGCGATTCCG

AGACCCACAACAATCAGATGTTTAAGGACAAGGCCACCCTGACAGTGGATAAGAGC

TCCAATACCGCCTACATGCACCTGTCTAGCCTGACATCTGAGGACAGCGCCGTGTAT

CACTGCGCCCGGCTGTACAGATGGTATTTTGACGTGTGGGGAGCAGGAACCACAGTG

ACCGTGTCCTCT (SEQ ID NO: 1)

QVQLQQPGAELVRPGASVKLSCKASGYTFTSYWMNWVKQRPGQGLEWIGMIDPSDSET

HNNQMFKDKATLTVDKSSNTAYMHLSSLTSEDSAVYHCARLYRWYFDVWGAGTTVTV

SS (SEQ ID NO: 2)

6F7 light chain variable region:

AACATCGTGATGACCCAGTCCCCCAAGTCTATGAGCATGTCCCTGGGCGAGAGGGTG

ACCCTGTCCTGTAAGGCCTCTGAGATCGTGGGCACATACGTGTCTTGGTTTCAGCAG

AAGCCACACCAGAGCCCCAAGCTGCTGATCTACGGCGCCTCCAATCGGTATACAGGC

GTGCCTGACAGATTCACCGGCTCTGGCAGCGCCACAGACTTCACCCTGACAATCTCT

AACGTGCAGGCCGAGGACCTGGCCGATTATCACTGCGGCCAGAGCTACAATTTCCCT

TATACCTTTGGCGGCGGCACAAAGCTGGAGATCAAG (SEQ ID NO: 3)

NIVMTQSPKSMSMSLGERVTLSCKASEIVGTYVSWFQQKPHQSPKLLIYGASNRYTGVP

DRFTGSGSATDFTLTISNVQAEDLADYHCGQSYNFPYTFGGGTKLEIK (SEQ ID NO: 4)

6F7CDR

HCDR1: GYTFTSYW (SEQ ID NO: 5)
HCDR2: IDPSDSET (SEQ ID NO: 6)
HCDR3: ARLYRWYFDV (SEQ ID NO: 7)
LCDR1: EIVGTY (SEQ ID NO: 8)
LCDR2: GAS (SEQ ID NO: 9)
LCDR3: GQSYNFPYT (SEQ ID NO: 10)

6F7H1VH:

CAGGTGCAGCTGGTGCAGAGCGGAGCAGAGGTGGTGAAGCCAGGAGCCTCTGTGAA GCTGAGCTGTAAGGCCTCCGGCTACACCTTCACAAGCTATTGGATGAACTGGGTGCG GCAGAGACCAGGACAGGGACTGGAGTGGATCGGAATGATCGACCCTTCCGATTCTG AGACCCACAATGCCCAGAAGTTTCAGGGCAAGGCCACCCTGACAGTGGACAAGAGC ACCTCCACAGCCTACATGCACCTGAGCTCCCTGCGGTCCGAGGACACAGCCGTGTAC TATTGCGCCAGGCTGTACCGCTGGTATTTTGACGTGTGGGGAGCAGGAACCACAGTG ACCGTGTCTAGC (SEQ ID NO: 11)

QVQLVQSGAEVVKPGASVKLSCKASGYTFTSYWMNWVRQRPGQGLEWIGMIDPSDSET HNAQKFQGKATLTVDKSTSTAYMHLSSLRSEDTAVYYCARLYRWYFDVWGAGTTVTV SS (SEQ ID NO: 12)

6F7L1VL:

AACATCGTGATGACCCAGTCCCCAGCCACAATGTCTATGAGCCCAGGAGAGAGGGT GACCCTGTCCTGTAGAGCCTCTGAGATCGTGGGCACATACGTGTCTTGGTTTCAGCA GAAGCCAGGACAGGCACCTAGGCTGCTGATCTACGGAGCAAGCAACAGGTATACCG GAGTGCCAGCACGCTTCTCCGGCTCTGGCAGCGGCACAGACTTTACCCTGACAATCA GCTCCGTGCAGCCTGAGGACCTGGCCGATTATCACTGCGGCCAGTCTTACAATTTCC CATATACCTTTGGCGGCGGCACAAAGCTGGAGATCAAG (SEQ ID NO: 13)

NIVMTQSPATMSMSPGERVTLSCRASEIVGTYVSWFQQKPGQAPRLLIYGASNRYTGVP ARFSGSGSGTDFTLTISSVQPEDLADYHCGQSYNFPYTFGGGTKLEIK (SEQ ID NO: 14)

6F7H2VH:

CAGGTGCAGCTGGTGCAGAGCGGAGCAGAGGTGGTGAAGCCAGGAGCCTCTGTGAA GGTGAGCTGTAAGGCCTCCGGCTACACCTTCACATCCTATTGGATGAACTGGGTGCG GCAGAGACCAGGACAGGGACTGGAGTGGATCGGAATCATCGACCCTTCCGATTCTG AGACCTCTAATGCCCAGAAGTTTCAGGGCCGGGTGACCCTGACAGTGGACAAGAGC ACCTCCACAGCCTACATGCACCTGAGCTCCCTGAGGAGCGAGGACACAGCCGTGTAC TATTGCGCCAGGCTGTACCGCTGGTATTTTGACGTGTGGGGAGCAGGAACCACAGTG ACCGTGTCTAGC (SEQ ID NO: 15)

QVQLVQSGAEVVKPGASVKVSCKASGYTFTSYWMNWVRQRPGQGLEWIGIIDPSDSET

SNAQKFQGRVTLTVDKSTSTAYMHLSSLRSEDTAVYYCARLYRWYFDVWGAGTTVTV

SS (SEQ ID NO: 16)

6F7L2VL:

AACATCGTGATGACCCAGTCCCCAGCCACACTGTCTCTGAGCCCAGGAGAGAGGGTG

ACCCTGTCCTGTAGAGCCTCTGAGATCGTGGGCACATACGTGTCTTGGTTTCAGCAG

AAGCCAGGACAGGCACCTAGGCTGCTGATCTATGGCGCCAGCAACAGGGCAACCGG

CATCCCCGCACGCTTCTCCGGCTCTGGCAGCGGCACAGACTTTACCCTGACAATCAG

CTCCCTGCAGCCTGAGGACCTGGCCGATTACTATTGCGGCCAGTCTTACAATTTCCCA

TATACCTTTGGCGGCGGCACAAAGCTGGAGATCAAG (SEQ ID NO: 17)

NIVMTQSPATLSLSPGERVTLSCRASEIVGTYVSWFQQKPGQAPRLLIYGASNRATGIPAR

FSGSGSGTDFTLTISSLQPEDLADYYCGQSYNFPYTFGGGTKLEIK (SEQ ID NO: 18)

6F7H3VH:

CAGGTGCAGCTGGTGCAGAGCGGAGCAGAGGTGGTGAAGCCAGGAGCCTCTGTGAA

GGTGAGCTGTAAGGCCTCCGGCTACACCTTCACATCCTATTGGATGAACTGGGTGCG

GCAGGCACCAGGACAGGGACTGGAGTGGATCGGCATCATCGACCCTTCCGATTCTGA

GACCTCTTACGCCCAGAAGTTTCAGGGCAGGGTGACCCTGACAGTGGACAAGAGCA

CCTCCACAGCCTATATGGAGCTGAGCTCCCTGCGCAGCGAGGACACAGCCGTGTACT

ATTGCGCCCGGCTGTACAGATGGTATTTTGACGTGTGGGGAGCAGGAACCACAGTGA

CCGTGTCTAGC (SEQ ID NO: 19)

QVQLVQSGAEVVKPGASVKVSCKASGYTFTSYWMNWVRQAPGQGLEWIGIIDPSDSET

SYAQKFQGRVTLTVDKSTSTAYMELSSLRSEDTAVYYCARLYRWYFDVWGAGTTVTVS

S (SEQ ID NO: 20)

6F7L3VL:

AACATCGTGATGACCCAGTCCCCAGCCACACTGTCTCTGAGCCCAGGAGAGAGGGTG

ACCCTGTCCTGTAGAGCCTCTGAGATCGTGGGCACATACCTGTCTTGGTATCAGCAG

AAGCCAGGACAGGCACCTAGGCTGCTGATCTACGGAGCCAGCACCAGGGCAACAGG

CATCCCCGCACGCTTCTCCGGCTCTGGCAGCGGCACCGACTTTACCCTGACAATCAG

CTCCCTGCAGCCTGAGGATTTTGCCGTGTACTATTGCGGCCAGTCTTACAATTTCCCA

TATACCTTTGGCGGCGGCACAAAGCTGGAGATCAAG (SEQ ID NO: 21)

NIVMTQSPATLSLSPGERVTLSCRASEIVGTYLSWYQQKPGQAPRLLIYGASTRATGIPAR

FSGSGSGTDFTLTISSLQPEDFAVYYCGQSYNFPYTFGGGTKLEIK (SEQ ID NO: 22)

6F7H4VH:

CAGGTGCAGCTGGTGCAGAGCGGAGCCGAAGTGGTGAAGCCTGGCGCCTCTGTGAA

GCTGTCCTGTAAGGCCTCTGGCTACACCTTTACATCCTACTGGATGAACTGGGTGCG

GCAGAGACCAGGCCAGTGCCTGGAGTGGATCGGCATGATCGACCCATCTGATTCCGA

GACCCACAATGCCCAGAAGTTTCAGGGCAAGGCCACACTGACCGTGGACAAGAGCA

CCTCCACAGCCTATATGCACCTGTCCTCTCTGAGAAGCGAGGATACAGCCGTGTATT

ACTGTGCCAGACTGTATCGGTGGTACTTTGACGTGTGGGGCGCCGGCACCACAGTGA

CCGTGTCTAGC (SEQ ID NO: 23)

QVQLVQSGAEVVKPGASVKLSCKASGYTFTSYWMNWVRQRPGQCLEWIGMIDPSDSET

HNAQKFQGKATLTVDKSTSTAYMHLSSLRSEDTAVYYCARLYRWYFDVWGAGTTVTV

SS (SEQ ID NO: 24)

6F7L4VL:

AATATCGTGATGACCCAGTCTCCTGCCACAATGTCTATGAGCCCTGGCGAGAGAGTG

ACCCTGTCTTGTAGGGCCAGCGAGATCGTGGGCACCTACGTGAGCTGGTTCCAGCAG

AAGCCAGGCCAGGCCCCAAGACTGCTGATCTATGGCGCCAGCAACCGGTATACAGG

CGTGCCTGCCAGATTTTCTGGCTCCGGCTCTGGCACCGATTTCACACTGACCATCTCT

AGCGTGCAGCCCGAGGATCTGGCCGATTACCACTGTGGCCAGTCCTACAACTTCCCC

TATACATTCGGGTGCGGCACTAAACTCGAGATCAAA (SEQ ID NO: 25)

NIVMTQSPATMSMSPGERVTLSCRASEIVGTYVSWFQQKPGQAPRLLIYGASNRYTGVP

ARFSGSGSGTDFTLTISSVQPEDLADYHCGQSYNFPYTFGCGTKLEIK (SEQ ID NO: 26)

8C5.1VH

GAAGTGCAGCTGGTTGAGAGCGGCGGAGGACTGGTGAAACCTGGAGGATCTCTGAA
GCTGAGCTGCGCTGCTTCCGGATTCACTTTTAGCAACTCCGCCGTGAGCTGGGTGAG
ACAAACACCTGAGAAGAGGCTGGAGTGGGTGGCTACAATCACAAGCGGCGTGAGCT
ACACCTACTACCTGGACTCCGTGAGGGGCAGATTCACAATCAGCAGGGACAACGCC
AAGAACACCCTGTACCTGCAGATGAGCAGCCTGAGGAGCGAGGATACCGCTATGTA
CTACTGTACCAGGCAGTTCAAGGGCAACGCCCTGGATTACTGGGGCCAAGGAACCTC
TGTGACAGTGAGCTCC(SEQ ID NO: 27)

EVQLVESGGGLVKPGGSLKLSCAASGFTFSNSAVSWVRQTPEKRLEWVATITSGVSYTY
YLDSVRGRFTISRDNAKNTLYLQMSSLRSEDTAMYYCTRQFKGNALDYWGQGTSVTVS
S (SEQ ID NO: 28)

8C5.1VL

GACATTGTGATGACCCAGTCCCCTTCCTCCCTGACAGTGACAGCTGGAGAGAAGGTG
ACAATGTCCTGCAAGAGCAGCCAGAGCCTGCTGAACTCCGGAAATCAGAAGAACTA
CCTGACCTGGTACCAGCAGAAGCCCGGACAACCACCTAAGCTGCTGATCTACTGGGC
CTCCACCAGGGAAAGCGGAGTTCCTGATAGATTCACCGGCAGCGGCTCCGGAACAG
ATTTCACACTGACAATCAACAACTTCCAGGCTGAGGACCTGGCCGTGTACTACTGTC
AGAACGACTACTTCTACCCTCTGACCTTCGGCGCCGGAACAAAGCTGGAACTGAAG
(SEQ ID NO: 29)

DIVMTQSPSSLTVTAGEKVTMSCKSSQSLLNSGNQKNYLTWYQQKPGQPPKLLIYWAST
RESGVPDRFTGSGSGTDFTLTINNFQAEDLAVYYCQNDYFYPLTFGAGTKLELK (SEQ ID
NO: 30)

8C5.1 CDR

HCDR1: GFTFSNSA (SEQ ID NO: 31)
HCDR2: ITSGVSYT (SEQ ID NO: 32)
HCDR3: TRQFKGNALDY (SEQ ID NO: 33)
LCDR1: QSLLNSGNQKNY (SEQ ID NO: 34)
LCDR2: WAS (SEQ ID NO: 35)
LCDR3: QNDYFYPLT (SEQ ID NO: 36)

8C5.1 H1VH

EVQLVESGGGLVKPGGSLRLSCAASGFTFSNSAVSWVRQAPGKRLEWVATITSGVSYTY YLDSVRGRFTISRDNAKNSLYLQMNSLRAEDTAVYYCTRQFKGNALDYWGQGTSVTVS S (SEQ ID NO: 37)

GAGGTGCAGCTGGTGGAGAGCGGCGGCGGCCTGGTGAAGCCCGGCGGCAGCCTGAG GCTGTCCTGCGCCGCCTCTGGCTTCACCTTTTCTAACAGCGCCGTGTCTTGGGTGAGG CAGGCACCTGGCAAGCGCCTGGAGTGGGTGGCCACCATCACATCCGGCGTGTCTTAC ACCTACTATCTGGACAGCGTGCGGGGCAGATTCACAATCAGCAGAGATAACGCCAA GAACAGCCTGTATCTGCAGATGAACTCTCTGCGGGCCGAGGACACCGCCGTGTACTA TTGTACAAGACAGTTTAAGGGCAATGCCCTGGATTACTGGGGCCAGGGCACCAGCGT GACAGTGAGCTCC (SEQ ID NO: 38)

8C5.1 H2VH

EVQLVESGGGLVKPGGSLRLSCAASGFTFSNSAVSWVRQAPGKGLEWVSTITSGVSYTY YADSVKGRFTISRDNAKNSLYLQMNSLRAEDTAVYYCTRQFKGNALDYWGQGTSVTVS S (SEQ ID NO: 39)

GAGGTGCAGCTGGTGGAGAGCGGCGGCGGCCTGGTGAAGCCCGGCGGCAGCCTGAG GCTGTCCTGCGCAGCATCTGGCTTCACCTTTTCTAACAGCGCCGTGTCTTGGGTGAGG CAGGCACCTGGCAAGGGACTGGAGTGGGTGAGCACCATCACATCCGGCGTGTCTTAC ACCTACTATGCCGACTCCGTGAAGGGCCGGTTCACAATCAGCAGAGATAACGCCAA GAACAGCCTGTATCTGCAGATGAACTCTCTGAGGGCCGAGGACACCGCCGTGTACTA TTGTACACGCCAGTTTAAGGGCAATGCCCTGGATTACTGGGGCCAGGGCACCAGCGT GACAGTGAGCTCC (SEQ ID NO: 40)

8C5.1 H3VH

EVQLVESGGGLVKPGGSLRLSCAASGFTFSNSAVSWVRQAPGKGLEWVSSITSGVSYTY YADSVKGRFTISRDNAKNSLYLQMNSLRAEDTAVYYCTRQFKGNALDYWGQGTSVTVS S (SEQ ID NO: 41)

GAGGTGCAGCTGGTGGAGAGCGGCGGCGGCCTGGTGAAGCCCGGCGGCAGCCTGAG GCTGTCCTGCGCAGCATCTGGCTTCACCTTTTCTAACAGCGCCGTGTCTTGGGTGAGG CAGGCACCTGGCAAGGGACTGGAGTGGGTGAGCTCCATCACATCCGGCGTGTCTTAC ACCTACTATGCCGACTCCGTGAAGGGCCGGTTCACAATCAGCAGAGATAACGCCAA GAACAGCCTGTATCTGCAGATGAACTCTCTGAGGGCCGAGGACACCGCCGTGTACTA TTGTACACGCCAGTTTAAGGGCAATGCCCTGGATTACTGGGGCCAGGGCACCAGCGT GACAGTGAGCTCC (SEQ ID NO: 42)

8C5.1 L1VL

DIVMTQSPSSLSVSPGERVTMSCKSSQSLLNSGNQKNYLTWYQQKPGQPPKLLIYWAST RESGVPDRFSGSGSGTDFTLTINSFQAEDVAVYYCQNDYFPLTFGAGTKLELK (SEQ ID NO: 43)

GATATCGTGATGACCCAGAGCCCATCTAGCCTGTCCGTGTCTCCAGGCGAGAGAGTG ACCATGAGCTGCAAGTCTTCCCAGAGCCTGCTGAATAGCGGCAACCAGAAGAACTA CCTGACATGGTACCAGCAGAAGCCAGGCCAGCCTCCAAAGCTGCTGATCTACTGGGC CAGCACAAGAGAGAGCGGCGTGCCAGACAGATTCTCTGGCAGCGGCTCTGGCACCG ACTTCACACTGACCATCAACTCCTTCCAGGCCGAGGATGTGGCCGTGTATTACTGCC AGAACGATTACTTCTATCCCCTGACATTCGGCGCCGGCACAAAGCTGGAGCTGAAG (SEQ ID NO: 44)

8C5.1 L2VL

DIVMTQSPSSLSVSPGERVTISCKSSQSLLNSGNQKNYLTWYQQKPGQPPKLLIYWASTR ESGVPDRFSGSGSGTDFTLTISSFQAEDVAVYYCQNDYFPLTFGAGTKLELK (SEQ ID NO: 45)

GACATCGTGATGACCCAGTCTCCAAGCTCCCTGAGCGTGTCCCAGGAGAGAGGGTG

ACAATCTCCTGCAAGTCTAGCCAGTCTCTGCTGAACAGCGGCAATCAGAAGAACTAC

CTGACCTGGTATCAGCAGAAGCCTGGCCAGCCCCCTAAGCTGCTGATCTACTGGGCC

TCCACCAGGGAGTCTGGAGTGCCAGACAGATTTTCTGGCAGCGGCTCCGGCACAGAT

TCACCCTGACAATCCTCTTTTCAGGCCGAGGACGTGGCCGTGTACTATTGTCAGA

ATGATTACTTCTATCCCCTGACCTTTGGCGCCGGCACAAAGCTGGAGCTGAAG   (SEQ

ID NO: 46)

8C5.1 L3VL

DIVMTQSPSSLSVSPGERVTISCKSSQSLLNSGNQKNYLAWYQQKPGQPPKLLIYWASTR

ESGVPDRFSGSGSGTDFTLTISSLQAEDVAVYYCQNDYFYPLTFGAGTKLELK  (SEQ   ID

NO: 47)

GACATCGTGATGACCCAGTCTCCAAGCTCCCTGAGCGTGTCCCCAGGAGAGAGGGTG

ACAATCTCCTGCAAGTCTAGCCAGTCTCTGCTGAACAGCGGCAATCAGAAGAACTAC

CTGGCCTGGTATCAGCAGAAGCCTGGCCAGCCCCCTAAGCTGCTGATCTACTGGGCC

TCCACCAGGGAGTCTGGAGTGCCAGACAGATTCTCTGGCAGCGGCTCCGGCACAGAC

TTCACCCTGACAATCCTCTCTGCAGGCCGAGGACGTGGCCGTGTACTATTGTCAG

AATGATTACTTCTATCCCCTGACCTTTGGCGCCGGCACAAAGCTGGAGCTGAAG (SEQ

ID NO: 48)

Amino acid sequence of TEV: ENLYFQG (SEQ ID NO: 49)

Amino acid sequence of Linker (GGGGS)4:
GGGGSGGGGSGGGGSGGGGS (SEQ ID NO: 50)

Amino acid sequence of the heavy chain constant region of human hG1WT

ASTKGPSVFPLAPSSKSTSGGTAALGCLVKDYFPEPVTVSWNSGALTSGVHTFPAVLQSS

GLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQY

NSTYRVVSVLTVLHQDWLNGKEYKCKVSNKALPAPIEKTISKAKGQPREPQVYTLPPSR

EEMTKNQVSLTCLVKGFYPSDIAVEWESNGQPENNYKTTPPVLDSDGSFFLYSKLTVDK

SRWQQGNVFSCSVMHEALHNHYTQKSLSLSPGK (SEQ ID NO: 61)

Hinge region, CH2 domain, and CH3 domain (without Knob mutation sites S354C and T366W):

EPKSSDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK    (SEQ
ID NO: 62)

Constant region sequence (without hole mutation sites Y349C, T366S, L368A, and Y407V):

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPG (SEQ ID NO: 63)

Amino acid sequence of the heavy chain of Hu5F9-G4

QVQLVQSGAEVKKPGASVKVSCKASGYTFTNYNMHWVRQAPGQRLEWMGTIYPGND
DTSYNQKFKDRVTITADTSASTAYMELSSLRSEDTAVYYCARGGYRAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK (SEQ ID NO: 64)

Amino acid sequence of the light chain of Hu5F9-G4

DIVMTQSPLSLPVTPGEPASISCRSSQSIVYSNGNTYLGWYLQKPGQSPQLLIYKVSNRFS
GVPDRFSGSGSGTDFTLKISRVEAEDVGVYYCFQGSHVPYTFGQGTKLEIKRTVAAPSVF
IFPPSDEQLKSGTASVVCLLNNFYPREAKVQWKVDNALQSGNSQESVTEQDSKDSTYSL
SSTLTLSKADYEKHKVYACEVTHQGLSSPVTKSFNRGEC (SEQ ID NO: 65)

DETAILED DESCRIPTION

Embodiments of the present invention will be described in detail below with reference to examples, but those skilled in the art will appreciate that the following examples are only for illustrating the present invention, and should not be construed as limitations to the scope of the present invention. Experimental procedures without specified conditions in the examples are conducted according to conventional conditions or conditions recommended by the manufacturer. Reagents or instruments used are all commercially available conventional products if the manufacturers thereof are not specified.

In the following experimental examples of the present invention, the isotype control antibodies used, i.e., hIgG1 and hIgG4, were antibodies targeting human anti-hen egg lysozyme (HEL), and the variable region sequences of these antibodies were from the study reported by Acierno et al., entitled "Affinity maturation increases the stability and plasticity of the Fv domain of anti-protein antibodies" (Acierno et al., J Mol Biol., 2007; 374(1): 130-46). In the constant region fragment of hIgG1, the heavy chain constant region is Ig gamma-1 chain C region, ACCESSION: P01857, and the light chain constant region is Ig kappa chain C region, ACCESSION: P01834. In hIgG4, the heavy chain constant region is Ig gamma-4 chain C region, ACCESSION: P01861.1 with S228P mutation introduced to improve the stability, and the light chain constant region is Ig kappa chain C region, ACCESSION: P01834. Both hIgG1 and hIgG4 were prepared by Akeso Biopharma Inc.

**Preparation Example 1: Preparation of Anti-human CD47 Antibody 6F7**

1. Preparation of hybridoma cell line LT012

The antigen used was CD47 IgV TEV-His (including human CD47 mature peptide of positions 19-141 of GenbankID: NP 942088.1 and TEV-his tag fusion protein) and 3T3-CD47 cells (NIH/3T3, manufacturer: ATCC, Cat. No.: CRL-1658; the cells were transfected with the human CD47 mature peptide on the basis of NIH/3T3 to construct a cell line stably expressing 3T3-CD47). Spleen cells of immunized mice were fused with myeloma cells of mice to prepare hybridoma cells. With CD47 IgV TEV-His and 3T3-CD47 cells separately taken as antigens, the hybridoma cells were screened by indirect ELISA to obtain hybridoma cells capable of secreting antibodies capable of specifically binding to CD47. The hybridoma cells obtained by ELISA screening were screened by competitive ELISA to obtain a hybridoma cell line capable of secreting a monoclonal antibody capable of competing for binding to human CD47 IgV TEV-His with the receptor human SIRPαECD-hFc-Biotin (SIRPαECD refers to the extracellular region of SIRPα, positions 31-373 of protein GenBank ACCESSION No. NP_542970.1; hFc refers to a human IgG Fc purification tag, specifically to Ig gamma-1 chain C region, positions 114-330 of GenbankID: P01857), which was then subjected to limiting dilution assay to obtain a stable hybridoma cell line. The hybridoma cell line above was named hybridoma cell line LT012, and the monoclonal antibody secreted by the cell line was named 6F7.

Hybridoma cell line LT012 was deposited at China Center for Type Culture Collection (CCTCC) on June 21, 2018 with a CCTCC designation of CCTCC NO. C2018135, the depository address being Wuhan University, Wuhan, China, postal code: 430072.

2. Preparation of anti-CD47 antibody 6F7

The cell line LT012 prepared above was cultured with a chemical defined medium (CD medium, containing 1% penicillin-streptomycin, Glutamax (Gibco 35050079)) in an incubator with 5% $CO_2$ at 37 °C. After 7 days, the supernatant was collected and subjected to high-speed centrifugation and vacuum filtration through a microfiltration membrane, and purified by using a HiTrap protein A HP column to obtain the antibody 6F7.

**Preparation Example 2: Sequence Analysis of Anti-CD47 Antibody 6F7**

mRNA was extracted from the cell line LT012 cultured in Preparation Example 1 according to the method described in the manual of RNAprep pure Cell/Bacteria Kit (Tiangen, Cat. No.: DP430).

cDNA was synthesized according to the manual of Invitrogen SuperScript® III First-Strand Synthesis System for RT-PCR kit and amplified by PCR.

The PCR-amplified products were directly subjected to TA cloning according to the manual of the pEASY-T1 Cloning Kit (Transgen CT101).

The TA cloning product was directly sequenced, and the sequencing results are as follows:

The nucleotide sequence of the heavy chain variable region is set forth in SEQ ID NO: 1 with a length of 351 bp.

The encoded amino acid sequence is set forth in SEQ ID NO: 2 with a length of 117 aa.

The nucleotide sequence of the light chain variable region is set forth in SEQ ID NO: 3 with a length of 321 bp.

The encoded amino acid sequence is set forth in SEQ ID NO: 4 with a length of 107 aa.

The 6 CDRs of antibody 6F7 are defined by the IMGT numbering system as follows:

The sequences of heavy chain HCDR1, HCDR2, and HCDR3 are set forth in SEQ ID NOs: 5, 6, and 7, respectively.

The sequences of light chain LCDR1, LCDR2, and LCDR3 are set forth in SEQ ID NOs: 8, 9, and 10, respectively.

**Preparation Example 3: Light and Heavy Chain Design and Preparation of Humanized Anti-Human CD47 Antibodies 6F7H1L1(hG4), 6F7H2L2(hG4), 6F7H3L3(hG4), and 6F7H4L4(hG4)**

1. Light and heavy chain design of humanized anti-human CD47 antibodies 6F7H1L1(hG4), 6F7H2L2(hG4), 6F7H3L3(hG4), and 6F7H4L4(hG4)

Based on the three-dimensional crystal structure of human CD47 protein (Hage T, Reinemer P, Sebald W., Crystals of a 1:1 Complex Between Human Interleukin-4 and the Extracellular Domain of Its Receptor Alpha Chain, Eur. J. Biochem., 1998; 258(2): 831-6.) and the sequence of antibody 6F7 obtained in Preparation Example 2, the variable region sequences of antibodies 6F7H1L1, 6F7H2L2, 6F7H3L3, and 6F7H4L4 were obtained by computer modeling and mutation design (antibody constant region sequences from NCBI database: the heavy chain constant region is Ig gamma-4 chain C region, ACCESSION: P01861.1; the light chain constant region is Ig kappa chain C region, ACCESSION: P01834).

The designed variable region sequences are as follows:

(1) Heavy and light chain variable region sequences of humanized monoclonal antibody 6F7H1L1

The nucleotide sequence of the heavy chain variable region is set forth in SEQ ID NO: 11 with a length of 351 bp.
The encoded amino acid sequence is set forth in SEQ ID NO: 12 with a length of 117 aa.
The nucleotide sequence of the light chain variable region is set forth in SEQ ID NO: 13 with a length of 321 bp.
The encoded amino acid sequence is set forth in SEQ ID NO: 14 with a length of 107 aa.
(2) Heavy and light chain variable region sequences of humanized monoclonal antibody 6F7H2L2 The nucleotide sequence of the heavy chain variable region is set forth in SEQ ID NO: 15 with a length of 351 bp.
The encoded amino acid sequence is set forth in SEQ ID NO: 16 with a length of 117 aa.
The nucleotide sequence of the light chain variable region is set forth in SEQ ID NO: 17 with a length of 321 bp.
The encoded amino acid sequence is set forth in SEQ ID NO: 18 with a length of 107 aa.
(3) Heavy and light chain variable region sequences of humanized monoclonal antibody 6F7H3L3 The nucleotide sequence of the heavy chain variable region is set forth in SEQ ID NO: 19 with a length of 351 bp.
The encoded amino acid sequence is set forth in SEQ ID NO: 20 with a length of 117 aa.
The nucleotide sequence of the light chain variable region is set forth in SEQ ID NO: 21 with a length of 321 bp.
The encoded amino acid sequence is set forth in SEQ ID NO: 22 with a length of 107 aa.
(4) Heavy and light chain variable region sequences of humanized monoclonal antibody 6F7H4L4 The nucleotide sequence of the heavy chain variable region is set forth in SEQ ID NO: 23 with a length of 351 bp.
The encoded amino acid sequence is set forth in SEQ ID NO: 24 with a length of 117 aa.
The nucleotide sequence of the light chain variable region is set forth in SEQ ID NO: 25 with a length of 321 bp.
The encoded amino acid sequence is set forth in SEQ ID NO: 26 with a length of 107 aa.
Moreover, 6F7H1L1, 6F7H2L2, 6F7H3L3, and 6F7H4L4 have identical HCDR1-HCDR3 and LCDR1-LCDR3 as follows:

the sequence of HCDR1 is set forth in SEQ ID NO: 5, the sequence of HCDR2 is set forth in SEQ ID NO: 6, and the sequence of HCDR3 is set forth in SEQ ID NO: 7;
the sequence of LCDR1 is set forth in SEQ ID NO: 8, the sequence of LCDR2 is set forth in SEQ ID NO: 9, and the sequence of LCDR3 is set forth in SEQ ID NO: 10.

**2. Preparation of humanized antibodies 6F7H1L1(hG4), 6F7H2L2(hG4), 6F7H3L3(hG4), and 6F7H4L4**

The heavy chain constant regions were all Ig gamma-4 chain C region, ACCESSION: P01861.1; the light chain constant regions were all Ig kappa chain C region, ACCESSION: P01834.
The heavy chain cDNA and light chain cDNA of 6F7H1L1(hG4), the heavy chain cDNA and light chain cDNA of 6F7H2L2(hG4), the heavy chain cDNA and light chain cDNA of 6F7H3L3(hG4), and the heavy chain cDNA and light chain cDNA of 6F7H4L4(hG4) were separately cloned into pUC57simple (provided by Genscript) vectors to obtain pUC57-simple-6F7H1 and pUC57simple-6F7L1, pUC57simple-6F7H2 and pUC57simple-6F7L2, pUC57simple-6F7H3 and pUC57simple-6F7L3, and pUC57simple-6F7H4 and pUC57simple-6F7L4, respectively. With reference to the standard techniques described in Molecular Cloning: A Laboratory Manual (Second Edition), the heavy and light chain full-length genes synthesized by EcoRI&HindIII digestion of genes were subcloned into an expression vector pcDNA3.1 through digestion by a restriction enzyme (EcoRI&HindIII) to obtain expression plasmids pcDNA3.1-6F7H1, pcDNA3.1-6F7L1, pcDNA3.1-6F7H2, pcDNA3.1-6F7L2, pcDNA3.1-6F7H3, pcDNA3.1-6F7L3, pcDNA3.1-6F7H4, and pcDNA3.1-6F7L4, and the heavy and light chain genes of the recombinant expression plasmids were further sequenced. Subsequently, the designed recombinant plasmid gene combinations containing the corresponding light and heavy chains (pcDNA3.1-6F7H1/pcDNA3.1-6F7L1, pcDNA3.1-6F7H2/pcDNA3.1-6F7L2, pcDNA3.1-6F7H3/pcDNA3.1-6F7L3, and pcDNA3.1-6F7H4/pcDNA3.1-6F7L4) were separately co-transfected into 293F cells, and then the culture solutions were collected and purified. After the sequences were verified, endotoxin-free expression plasmids were prepared and were transiently transfected into HEK293 cells for antibody expression. The cell cultures were collected after 7 days of culture, and subjected to affinity purification on a Protein A column (MabSelect SURE (GE)) to obtain humanized antibodies.

## Preparation Example 4: Preparation of Anti-CLDN18.2 Antibody 8C5.1

1. Preparation of hybridoma cell line LT020

The immunogen used was a 3T3 cell line (Chinese Academy of Sciences) overexpressing human Claudin18.2 (GenbankID: NP_001002026.1) (3T3 hClaudin18.2). Spleen cells of immunized mice were fused with myeloma cells of mice to prepare hybridoma cells. With a CHO-K1 cell line overexpressing human Claudin18.2 (Institute of Basic Medical Sciences, Chinese Academy of Medical Sciences) (CHO-K1-hClaudin18.2) as an antigen, the hybridoma cells were screened by indirect ELISA to obtain hybridoma cells capable of secreting antibodies specifically binding to CLDN18.2. The hybridoma cells obtained by screening were subjected to limiting dilution assay to obtain a stable hybridoma cell line. The hybridoma cell line above was named hybridoma cell line LT020, and the monoclonal antibody secreted by the hybridoma cell line was named 8C5.1. Hybridoma cell line LT020 was deposited at China Center for Type Culture Collection (CCTCC) on May 19, 2022 with a CCTCC designation of CCTCC NO. C2022124, the depository address being Wuhan University, Wuhan, China, postal code: 430072.

2. Preparation of anti-CLDN18.2 antibody 8C5.1

The cell line LT020 prepared above was cultured in a CD Medium (Chemical Defined Medium, containing 4% Glutamax (Gibco 35050079) and 1% penicillin-streptomycin (Gibco 15140163)) at 5% $CO_2$ and 37 °C. After 7 days, the cell culture supernatant was collected, subjected to high-speed centrifugation and vacuum filtration through a microfiltration membrane, and purified by using a HiTrap protein A HP column to obtain the antibody 8C5.1.

## Preparation Example 5: Sequence Analysis of Anti-CLDN18.2 Antibody 8C5.1

mRNA was extracted from the cell line LT020 cultured in Preparation Example 4 according to the method described in the manual of RNAprep pure Cell/Bacteria Kit (Tiangen, Cat. No.: DP430).
cDNA was synthesized according to the manual of Invitrogen SuperScript® III First-Strand Synthesis System for RT-PCR kit and amplified by PCR.
The PCR-amplified product was directly subjected to TA cloning according to the manual of the pEASY-T1 Cloning Kit (Transgen CT101).
The TA cloning product was directly sequenced, and the sequencing results are as follows:
The nucleotide sequence of the heavy chain variable region is set forth in SEQ ID NO: 27 with a length of 354 bp.
The encoded amino acid sequence is set forth in SEQ ID NO: 28 with a length of 118 amino acids. The nucleotide sequence of the light chain variable region is set forth in SEQ ID NO: 29 with a length of 339 bp.
The encoded amino acid sequence is set forth in SEQ ID NO: 30 with a length of 113 amino acids. The 6 CDRs of the antibody 8C5.1 defined by the IMGT numbering system are as follows:
The sequences of heavy chain HCDR1, HCDR2, and HCDR3 are set forth in SEQ ID NOs: 31, 32, and 33, respectively.
The sequences of light chain LCDR1, LCDR2, and LCDR3 are set forth in SEQ ID NOs: 34, 35, and 36, respectively.

## Preparation Example 6: Light and Heavy Chain Design and Preparation of Humanized Anti-Human CLDN18.2 Antibodies

1. Light and heavy chain design of humanized anti-human CLDN18.2 antibodies 8C5.1H1L1, 8C5.1H1L2, 8C5.1H2L1, 8C5.1H2L2, 8C5.1H2L3, 8C5.1H3L2, and 8C5.1H3L3

Based on the three-dimensional crystal structure of human CLDN18.2 protein and the sequence of antibody 8C5 obtained in Preparation Example 5, the variable region sequences of antibodies 8C5.1H1L1, 8C5.1H1L2, 8C5.1H2L1, 8C5.1H2L2, 8C5.1H2L3, 8C5.1H3L2, and 8C5.1H3L3 were obtained by computer modeling and then mutation design (antibody constant region sequences from NCBI database: the heavy chain constant region is Ig gamma-1 chain C region, SEQ ID NO: 61; the light chain constant region is Ig kappa chain C region, ACCESSION: P01834). The designed variable region sequences are shown in Table A below.

| No. | Name of antibody | Amino acid sequence of heavy chain variable region SEQ ID NO: | Nucleotide sequence of heavy chain variable region SEQ ID NO: | Amino acid sequence of light chain variable region SEQ ID NO: | Nucleotide sequence of light chain variable region SEQ ID NO: |
|---|---|---|---|---|---|
| 1 | 8C5.1H1L1 | 37 | 38 | 43 | 44 |
| 2 | 8C5.1H1L2 | 37 | 38 | 45 | 46 |
| 3 | 8C5.1H2L1 | 39 | 40 | 43 | 44 |
| 4 | 8C5.1H2L2 | 39 | 40 | 45 | 46 |
| 5 | 8C5.1H2L3 | 39 | 40 | 47 | 48 |
| 6 | 8C5.1H3L2 | 41 | 42 | 45 | 46 |
| 7 | 8C5.1H3L3 | 41 | 42 | 47 | 48 |

For each of the above 7 antibodies 8C5.1H1L1, 8C5.1H1L2, 8C5.1H2L1, 8C5.1H2L2, 8C5.1H2L3, 8C5.1H3L2, and 8C5.1H3L3, the length of the nucleotide sequence of the heavy chain variable region was 354 bp and the length of the encoded amino acid sequence was 118 aa, and the length of the nucleotide sequence of the light chain variable region was 339 bp and the length of the encoded amino acid sequence was 113 aa.

Besides, the above 7 antibodies had the same HCDR1-HCDR3 and LCDR1-LCDR3 shown below: The sequences of HCDR1, HCDR2, and HCDR3 are set forth in SEQ ID NOs: 31, 32, and 22, respectively.

The sequences of LCDR1, LCDR2, and LCDR3 are set forth in SEQ ID NOs: 34, 35, and 36, respectively.

**2. Preparation of humanized antibodies 8C5.1H1L1, 8C5.1H1L2, 8C5.1H2L1,** 8C5.1H2L2, 8C5.1H2L3, 8C5.1H3L2, and 8C5.1H3L3

The heavy chain constant regions were all Ig gamma-1 chain C region, SEQ ID NO: 61; the light chain constant regions were all Ig kappa chain C region, ACCESSION: P01834.

The heavy chain cDNA and light chain cDNA of each of 8C5.1H1L1, 8C5.1H1L2, 8C5.1H2L1, 8C5.1H2L2, 8C5.1H2L3, 8C5.1H3L2, and 8C5.1H3L3 were cloned into a pUC57simple (provided by GenScript) vector to respectively obtain: pUC57simple-8C5.1H1, pUC57simple-8C5.1L1;

pUC57simple-8C5.1H1, pUC57simple-8C5.1L2;
pUC57simple-8C5.1H2, pUC57simple-8C5.1L1;
pUC57simple-8C5.1H2, pUC57simple-8C5.1L2;
pUC57simple-8C5.1H2, pUC57simple-8C5.1L3;
pUC57simple-8C5.1H3, pUC57simple-8C5.1L2; and
pUC57simple-8C5.1H3, pUC57simple-8C5.1L3.

According to the standard techniques described in Molecular Cloning: A Laboratory Manual (Second Edition), EcoRI&HindIII was used to digest the synthesized full-length heavy and light chain genes. The genes were subcloned into the expression vector pcDNA3.1 by the digestion of the restriction enzyme (EcoRI&HindIII) to obtain expression plasmids pcDNA3.1-8C5.1H1, pcDNA3.1-8C5.1L1, pcDNA3.1-8C5.1H2, pcDNA3.1-8C5.1L2, pcDNA3.1-8C5.1H3, and pcDNA3.1-8C5.1L3, and further the heavy/light chain genes of the recombinant expression plasmids were sequenced. Then the designed gene combinations comprising the corresponding light and heavy chain recombinant plasmids (pcDNA3.1-8C5.1H1/pcDNA3.1-8C5.1L1,

pcDNA3.1-8C5.1H1/pcDNA3.1-8C5.1L2,
pcDNA3.1-8C5.1H2/pcDNA3.1-8C5.1L1,
pcDNA3.1-8C5.1H2/pcDNA3.1-8C5.1L2,
pcDNA3.1-8C5.1H2/pcDNA3.1-8C5.1L3,
pcDNA3.1-8C5.1H3/pcDNA3.1-8C5.1L2, and
pcDNA3.1-8C5.1H3/pcDNA3.1-8C5.1L3) were each co-transfected into 293F cells, and then the culture solutions were collected and purified. After the sequences were verified to be correct by sequencing, endotoxin-free expression plasmids were prepared and transiently transfected into HEK293 cells for antibody expression. After 7 days, the cell culture solutions were collected and subjected to affinity purification on a Protein A column to obtain humanized antibodies.

**Preparation Example 7: Design and Preparation of Anti-CLDN18.2/Anti-CD47 Bispecific Antibody**

1. Structural design

The bispecific antibody in this example was named AsAb-8C4703, the structure of which is shown in Table 1 below.

Table 1: Composition design of heavy and light chains of bispecific antibody

| Peptide chain name | Structural composition |
|---|---|
| HC1 | ScFv (6F7H4VH + Linker + 6F7L4VL) + hinge region + Fc Knob (hIgG1 CH2 and hIgG1 CH3, carrying mutations S354C and T366W) |
| HC2 | 8C5.1H3VH + hIgG1 constant region Hole (hIgG1, carrying mutations Y349C, T366S, L368A, and Y407V) |
| L3 | 8C5.1L3VL + human Ig kappa constant region |

In Table 1 above:
The linker fragment, i.e., Linker, has an amino acid sequence set forth in SEQ ID NO: 50.

The mutation positions of Knob and Hole are numbered according to EU numbering system.

The bispecific antibody AsAb-8C4703 comprises three peptide chains from left to right, named HC1, HC2, and L3, respectively, the composition of which is as follows:

(1) HC1 composition: ScFv (VH of 6F7H4 + linker sequence + VL of 6F7L4) + hinge region + hIgG1 CH2 + hIgG1 CH3, which contains Knob mutation sites (serine at position 354 mutated to cysteine S354C and threonine at position 366 mutated to tryptophan T366W).

The amino acid sequence of the scFv sequence is as follows (where the underlining is the Linker sequence):

QVQLVQSGAEVVKPGASVKLSCKASGYTFTSYWMNWVRQRPGQCLEWIGMIDPSDSET

HNAQKFQGKATLTVDKSTSTAYMHLSSLRSEDTAVYYCARLYRWYFDVWGAGTTVTV

SS<u>GGGGSGGGGSGGGGSGGGGS</u>NIVMTQSPATMSMSPGERVTLSCRASEIVGTYVSWFQ

QKPGQAPRLLIYGASNRYTGVPARFSGSGSGTDFTLTISSVQPEDLADYHCGQSYNFPYT

FGCGTKLEIK (SEQ ID NO: 51)

Hinge region, CH2 domain, and CH3 domain (where the double underlining is the Knob mutation sites S354C and T366W):

EPKSSDKTHTCPPCPAPELLGGPSVFLFPPKPKDTLMISRTPEVTCVVVDVSHEDPEVKFN

WYVDGVEVHNAKTKPREEQYNSTYRVVSVLTVLHQDWLNGKEYKCKVSNKALPAPIE

KTISKAKGQPREPQVYTLPP<u>C</u>RDELTKNQVSL<u>W</u>CLVKGFYPSDIAVEWESNGQPENNYK

TTPPVLDSDGSFFLYSKLTVDKSRWQQGNVFSCSVMHEALHNHYTQKSLSLSPGK  (SEQ

ID NO: 52)

The full-length amino acid sequence of HC1 is as follows: (476 aa, where the underlining is the Linker sequence and the double underlining is the Knob mutation site)

QVQLVQSGAEVVKPGASVKLSCKASGYTFTSYWMNWVRQRPGQCLEWIGMIDPSDSET

HNAQKFQGKATLTVDKSTSTAYMHLSSLRSEDTAVYYCARLYRWYFDVWGAGTTVTV

SSGGGGSGGGGSGGGGSGGGGSNIVMTQSPATMSMSPGERVTLSCRASEIVGTYVSWFQ

QKPGQAPRLLIYGASNRYTGVPARFSGSGSGTDFTLTISSVQPEDLADYHCGQSYNFPYT

FGCGTKLEIKEPKSSDKTHTCPPCPAPELLGGPSVFLFPPKPKDTLMISRTPEVTCVVVDV

SHEDPEVKFNWYVDGVEVHNAKTKPREEQYNSTYRVVSVLTVLHQDWLNGKEYKCKV

SNKALPAPIEKTISKAKGQPREPQVYTLPPCRDELTKNQVSLWCLVKGFYPSDIAVEWES

NGQPENNYKTTPPVLDSDGSFFLYSKLTVDKSRWQQGNVFSCSVMHEALHNHYTQKSL

SLSPGK (SEQ ID NO: 53)

The full-length nucleotide sequence of HC1 is as follows:

CAGGTGCAGCTGGTGCAGAGCGGAGCCGAAGTGGTGAAGCCTGGCGCCTCTGTGAA

GCTGTCCTGTAAGGCCTCTGGCTACACCTTTACATCCTACTGGATGAACTGGGTGCG

GCAGAGACCAGGCCAGTGCCTGGAGTGGATCGGCATGATCGACCCATCTGATTCCGA

GACCCACAATGCCCAGAAGTTTCAGGGCAAGGCCACACTGACCGTGGACAAGAGCA

CCTCCACAGCCTATATGCACCTGTCCTCTCTGAGAAGCGAGGATACAGCCGTGTATT

ACTGTGCCAGACTGTATCGGTGGTACTTTGACGTGTGGGGCGCCGGCACCACAGTGA

CCGTGTCTAGCGGAGGCGGAGGATCTGGAGGAGGAGGCAGTGGCGGAGGGGGAAG

CGGCGGAGGAGGATCAAATATCGTGATGACCCAGTCTCCTGCCACAATGTCTATGAG

CCCTGGCGAGAGAGTGACCCTGTCTTGTAGGGCCAGCGAGATCGTGGGCACCTACGT

GAGCTGGTTCCAGCAGAAGCCAGGCCAGGCCCCAAGACTGCTGATCTATGGCGCCA

GCAACCGGTATACAGGCGTGCCTGCCAGATTTTCTGGCTCCGGCTCTGGCACCGATT

TCACACTGACCATCTCTAGCGTGCAGCCCGAGGATCTGGCCGATTACCACTGTGGCC

AGTCCTACAACTTCCCCTATACATTCGGGTGCGGCACTAAACTCGAGATCAAAGAGC

CCAAATCCTCCGACAAGACACACCTGTCCCCCCTGTCCTGCTCCCGAACTCCTCG

GAGGGCCCTCCGTGTTTCTGTTCCCTCCAAAGCCTAAAGATACTCTGATGATTAGTAG

GACTCCCGAAGTCACCTGTGTGGTCGTGGACGTGTCACACGAAGATCCTGAGGTCAA

ATTCAACTGGTACGTGGACGGCGTCGAGGTGCATAATGCCAAGACCAAGCCCCGCG

AGGAACAGTACAACAGCACCTATCGAGTCGTGTCCGTCCTGACAGTGCTGCACCAGG

ATTGGCTGAACGGGAAGGAGTATAAGTGCAAAGTGAGCAATAAGGCTCTGCCCGCA

CCTATCGAGAAGACCATTTCCAAGGCTAAAGGACAGCCCCGCGAGCCTCAGGTGTAC

ACACTGCCCCCTTGCCGGGACGAACTGACCAAGAACCAGGTCTCCCTGTGGTGTCTG

GTGAAGGGCTTCTACCCAAGCGATATCGCCGTGGAGTGGGAATCCAATGGACAGCC

CGAGAACAATTACAAGACCACCACCCGTGCTGGACTCCGATGGCAGCTTCTTCCT

GTATTCCAAGCTGACAGTGGACAAGAGCCGGTGGCAGCAGGGGAACGTCTTCAGTT

GCTCAGTGATGCACGAGGCCCTGCACAATCATTACACTCAGAAGAGCCTGTCCCTGT

CTCCAGGCAAA (SEQ ID NO: 54)

(2) HC2 composition: VH of 8C5.1H3 + constant region of hIgG1, which contains Hole mutation sites (tyrosine at position 349 mutated to cysteine Y349C, threonine at position 366 mutated to serine T366S, leucine at position 368 mutated to alanine L368A, and tyrosine at position 407 mutated to valine Y407V)
The amino acid sequence of 8C5.1H3VH is set forth in SEQ ID NO: 41.
Constant region sequence (where the double underlining is the Hole mutation sites Y349C, T366S, L368A, and Y407V):

ASTKGPSVFPLAPSSKSTSGGTAALGCLVKDYFPEPVTVSWNSGALTSGVHTFPAVLQSS

GLYSLSSVVTVPSSSLGTQTYICNVNHKPSNTKVDKKVEPKSCDKTHTCPPCPAPELLGG

PSVFLFPPKPKDTLMISRTPEVTCVVVDVSHEDPEVKFNWYVDGVEVHNAKTKPREEQY

NSTYRVVSVLTVLHQDWLNGKEYKCKVSNKALPAPIEKTISKAKGQPREPQV<u>C</u>TLPPSR

DELTKNQVSL<u>S</u><u>C</u><u>A</u>VKGFYPSDIAVEWESNGQPENNYKTTPPVLDSDGSFFL<u>V</u>SKLTVDKS

RWQQGNVFSCSVMHEALHNHYTQKSLSLSPG (SEQ ID NO: 55)

The full-length amino acid sequence of HC2 is as follows: (447 aa, where the double underlining is the Hole mutation site)

EVQLVESGGGLVKPGGSLRLSCAASGFTFSNSAVSWVRQAPGKGLEWVSSITSGVSYTY

YADSVKGRFTISRDNAKNSLYLQMNSLRAEDTAVYYCTRQFKGNALDYWGQGTSVTVS

SASTKGPSVFPLAPSSKSTSGGTAALGCLVKDYFPEPVTVSWNSGALTSGVHTFPAVLQS

SGLYSLSSVVTVPSSSLGTQTYICNVNHKPSNTKVDKKVEPKSCDKTHTCPPCPAPELLG

GPSVFLFPPKPKDTLMISRTPEVTCVVVDVSHEDPEVKFNWYVDGVEVHNAKTKPREEQ

YNSTYRVVSVLTVLHQDWLNGKEYKCKVSNKALPAPIEKTISKAKGQPREPQV<u>C</u>TLPPS

RDELTKNQVSL<u>S</u><u>C</u><u>A</u>VKGFYPSDIAVEWESNGQPENNYKTTPPVLDSDGSFFL<u>V</u>SKLTVD

KSRWQQGNVFSCSVMHEALHNHYTQKSLSLSPG (SEQ ID NO: 56)

The full-length nucleotide sequence of HC2 is as follows:

GAGGTGCAGCTGGTGGAGAGCGGCGGCGGCCTGGTGAAGCCCGGCGGCAGCCTGAG

GCTGTCCTGCGCAGCATCTGGCTTCACCTTTTCTAACAGCGCCGTGTCTTGGGTGAGG

CAGGCACCTGGCAAGGGACTGGAGTGGGTGAGCTCCATCACATCCGGCGTGTCTTAC

ACCTACTATGCCGACTCCGTGAAGGGCCGGTTCACAATCAGCAGAGATAACGCCAA

GAACAGCCTGTATCTGCAGATGAACTCTCTGAGGGCCGAGGACACCGCCGTGTACTA

TTGTACACGCCAGTTTAAGGGCAATGCCCTGGATTACTGGGGCCAGGGCACCAGCGT

GACAGTGAGCTCCGCCTCCACAAAGGGGCCCAGCGTGTTCCTCTCGCCCCCTCCTC

CAAAAGCACCAGCGGAGGAACCGCTGCTCTCGGATGTCTGGTGAAGGACTACTTCCC

TGAACCCGTCACCGTGAGCTGGAATAGCGGCGCTCTGACAAGCGGAGTCCATACATT

CCCTGCTGTGCTGCAAAGCAGCGGACTCTATTCCCTGTCCAGCGTCGTCACAGTGCC

CAGCAGCAGCCTGGGCACCCAGACCTACATCTGTAACGTCAACCACAAGCCCTCCAA

CACCAAGGTGGACAAGAAAGTGGAGCCCAAATCCTGCGACAAGACCCATACCTGCC

CACCTTGTCCAGCTCCAGAGCTGCTGGGAGGACCAAGCGTGTTCCTGTTTCCACCCA

AGCCTAAAGACACCCTGATGATCAGCAGAACCCCAGAGGTCACATGTGTGGTCGTG

GACGTGTCCCACGAGGACCCCGAAGTCAAGTTTAACTGGTACGTGGATGGCGTGGA

GGTCCATAATGCCAAGACCAAGCCCCGCGAAGAGCAGTACAACTCAACTTATCGAG

TCGTGAGCGTGCTGACCGTCCTGCACCAGGACTGGCTGAACGGAAAGGAGTATAAG

TGCAAAGTGTCTAACAAGGCCCTGCCTGCTCCAATCGAGAAGACCATTAGCAAGGCC

AAAGGCCAGCCCAGAGAACCTCAGGTGTGCACCCTGCCTCCAAGCAGGGATGAGCT

GACAAAGAACCAGGTCAGCCTGTCCTGTGCTGTGAAAGGATTCTACCCCTCCGACAT

CGCAGTGGAGTGGGAATCTAATGGCCAGCCTGAGAACAATTATAAGACCACACCCC

CTGTGCTGGATTCAGACGGCAGCTTCTTCCTGGTGAGCAAGCTGACTGTGGATAAGT

CAAGGTGGCAGCAGGGCAACGTGTTCAGCTGTAGTGTGATGCACGAGGCCCTGCAC

AATCATTACACCCAGAAATCACTGAGCCTGTCCCCGGG (SEQ ID NO: 57)

(3) L3 composition: VL of 8C5.1L3 + human Ig kappa constant region
The amino acid sequence of 8C5.1L3VL is set forth in SEQ ID NO: 47.
Amino acid sequence of human Ig kappa constant region

RTVAAPSVFIFPPSDEQLKSGTASVVCLLNNFYPREAKVQWKVDNALQSGNSQESVTEQ

DSKDSTYSLSSTLTLSKADYEKHKVYACEVTHQGLSSPVTKSFNRGEC (SEQ ID NO: 58)

The full-length amino acid sequence of L3 is as follows:

DIVMTQSPSSLSVSPGERVTISCKSSQSLLNSGNQKNYLAWYQQKPGQPPKLLIYWASTR

ESGVPDRFSGSGSGTDFTLTISSLQAEDVAVYYCQNDYFYPLTFGAGTKLELKRTVAAPS

VFIFPPSDEQLKSGTASVVCLLNNFYPREAKVQWKVDNALQSGNSQESVTEQDSKDSTY

SLSSTLTLSKADYEKHKVYACEVTHQGLSSPVTKSFNRGEC (SEQ ID NO: 59)

The full-length nucleotide sequence of L3 is as follows:

GACATCGTGATGACCCAGTCTCCAAGCTCCCTGAGCGTGTCCCCAGGAGAGAGGGTG

ACAATCTCCTGCAAGTCTAGCCAGTCTCTGCTGAACAGCGGCAATCAGAAGAACTAC

CTGGCCTGGTATCAGCAGAAGCCTGGCCAGCCCCCTAAGCTGCTGATCTACTGGGCC

TCCACCAGGGAGTCTGGAGTGCCAGACAGATTCTCTGGCAGCGGCTCCGGCACAGAC

TTCACCCTGACAATCTCCTCTCTGCAGGCCGAGGACGTGGCCGTGTACTATTGTCAG

AATGATTACTTCTATCCCCTGACCTTTGGCGCCGGCACAAAGCTGGAGCTGAAGCGT

ACGGTGGCAGCCCCATCTGTCTTCATTTTTCCCCCTAGTGACGAGCAGCTGAAATCCG

GAACAGCCTCTGTGGTCTGTCTGCTGAACAATTTCTACCCTCGCGAAGCCAAGGTGC

AGTGGAAAGTCGATAACGCTCTGCAGAGTGGCAATTCACAGGAGAGCGTGACTGAA

CAGGACTCCAAGGATTCTACCTATAGTCTGAGCTCCACTCTGACCCTGTCCAAAGCA

GATTACGAAAGCACAAAGTGTATGCCTGTGAGGTCACCCACCAGGGGCTGAGTTCT

CCAGTCACCAAATCCTTCAACAGAGGCGAATGT (SEQ ID NO: 60)

The peptide chains HC1 and HC2 of the bispecific antibody AsAb-8C4703 are linked by two disulfide bonds of the hinge region, and HC2 and L3 are linked by one disulfide bond.

2. Molecular construction and expression and purification of antibodies

The cDNA sequences encoding the three polypeptide chains of bispecific antibody AsAb-8C4703 were separately cloned into pUC57simple (provided by Genscript) vectors to obtain plasmids pUC57simple-HC1, pUC57-simple-HC2, and pUC57simple-L3, respectively.

Plasmids pUC57simple-HC1, pUC57simple-HC2, and pUC57simple-L3 were each digested by an enzyme (HindIII&EcoRI), and after recovery by electrophoresis, were subcloned into pcDNA3.1 vectors. The recombinant plasmids were extracted and co-transfected into 293F cells. After 7 days of cell culture, the culture solution was centrifuged at high speed. The supernatant was concentrated and loaded onto a HiTrap MabSelect SuRe column. The protein was eluted in one step with an elution buffer. The target sample was isolated, and the buffer was exchanged into PBS.

The anti-CLDN18.2/anti-CD47 bispecific antibody prepared is used in the following experiments.

**Example 1: FACS Assay of Binding Activity of Anti-CLDN18.2/Anti-CD47 Bispecific Antibody**

1. FACS (fluorescence activated cell sorter) assay of binding activity of anti-CLDN18.2/anti-CD47 bispecific antibody to CHO-K1-CLDN18.2-CD47 cells

CHO-K1-CLDN18.2-CD47 cells (CHO-K1 cells overexpress CLDN18.2 and CD47 antigens, and are obtained from Cell Resource Center, Institute of Basic Medical Sciences, Chinese Academy of Medical Sciences) in the logarithmic growth phase were collected, and were transferred to a V-bottomed 96-well plate at $1 \times 10^5$ cells/well. 100 $\mu$L of 1% PBSA

(PBS + 1% BSA) was added, and the mixture was centrifuged at 1000× g for 5 min, followed by removal of the supernatant. 100 μL of antibodies diluted with 1% PBSA (at final concentrations of 900 nM, 300 nM, 100 nM, 33.3 nM, 11.1 nM, 3.7 nM, 1.23 nM, 0.41 nM, 0.041 nM, and 0.0041 nM, respectively) were added. The mixture was mixed gently and well, and then incubated on ice for 40 min. 150 μL of 1% PBSA was added to each well, and the mixture was centrifuged at 1000× g for 5 min. The supernatant was discarded, and the plate was further washed three times at 200 μL/time. 300-fold diluted PE anti-human IgG Fc antibody (Biolegend, Cat. 409304) was added for resuspending, and the mixture was well mixed. The diluted antibody was added to corresponding samples at 100 μL/well, and the cell pellet was resuspended and incubated on ice in the dark for 0.5 h. 150 μL of 1% PBSA was added to each well, and the mixture was centrifuged at 1000 g for 5 min, followed by removal of the supernatant. Then the plate was washed twice with 200 μL of 1% PBSA. 200 μL of 1% PBSA was added to each well to resuspend the cell pellet, and the mixture was transferred to a flow cytometry tube. FACS Calibur assay was performed, and the binding activity was analyzed.

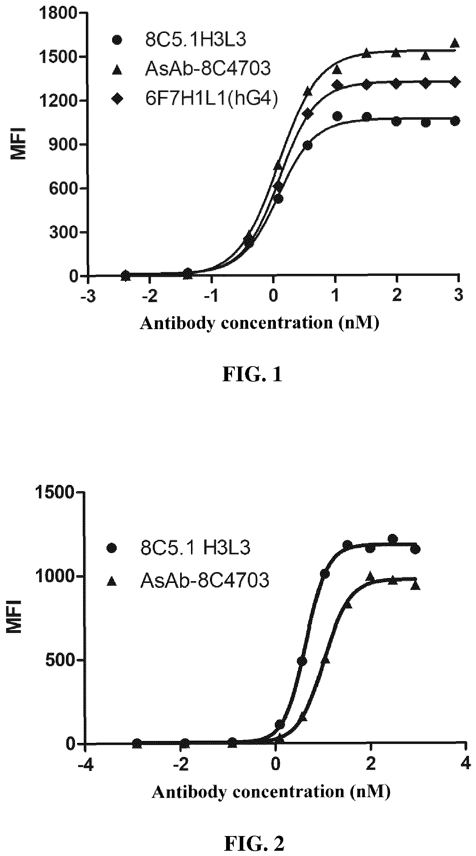

The experimental results are shown in Table 2 and FIG. 1.

Table 2: FACS assay of binding activity of 8C5.1H3L3, 6F7H1L1(hG4), and AsAb-8C4703 to CHO-K1-CLDN18.2-CD47 cells

|  | 8C5.1H3L3 | 6F7H1L1(hG4) | AsAb-8C4703 |
|---|---|---|---|
| $EC_{50}$ (nM) | 1.220 | 1.295 | 1.245 |

The results showed that under the same experimental conditions, the $EC_{50}$ of 8C5.1H3L3, 6F7H1L1(hG4), and AsAb-8C4703 for binding to CHO-K1-CLDN18.2-CD47 cells was 1.220 nM, 1.295 nM, and 1.245 nM, respectively.

The results show that under the same experimental conditions, 8C5.1H3L3, 6F7H1L1(hG4), and AsAb-8C4703 exhibit comparable binding activity to CHO-K1-CLDN18.2-CD47 cells, suggesting that AsAb-8C4703 has the activity of effectively binding to cells expressing both CLDN18.2 and CD47.

2. FACS assay of binding activity of anti-CLDN18.2/anti-CD47 bispecific antibody to CHO-K1-CLDN18.2 cells

CHO-K1-CLDN18.2 cells (CHO-K1 cells overexpress CLDN18.2 antigens, and are obtained from Cell Resource Center, Institute of Basic Medical Sciences, Chinese Academy of Medical Sciences) in the logarithmic growth phase were collected, and were transferred to a V-bottomed 96-well plate at $2.5 \times 10^5$ cells/well. 100 μL of 1% PBSA (PBS + 1% BSA) was added, and the mixture was centrifuged at 1000× g for 5 min, followed by removal of the supernatant. 100 μL of antibodies diluted with 1% PBSA (at final concentrations of 900 nM, 300 nM, 100 nM, 33.3 nM, 11.1 nM, 3.7 nM, 1.23 nM, 0.123 nM, 0.0123 nM, and 0.00123 nM, respectively) were added. The mixture was mixed gently and well, and then incubated on ice for 40 min. 150 μL of 1% PBSA was added to each well, and the mixture was centrifuged at 1000× g for 5 min. The supernatant was discarded, and the plate was further washed three times at 200 μL/time. 300-fold diluted PE anti-human IgG Fc antibody (Biolegend, Cat. 409304) was added for resuspending, and the mixture was well mixed. The diluted antibody was added to corresponding samples at 100 μL/well, and the cell pellet was resuspended and incubated on ice in the dark for 0.5 h. 150 μL of 1% PBSA was added to each well, and the mixture was centrifuged at 1000 g for 5 min, followed by removal of the supernatant. Then the plate was washed twice with 200 μL of 1% PBSA. 200 μL of 1% PBSA was added to each well to resuspend the cell pellet, and the mixture was transferred to a flow cytometry tube for FACS Calibur assay.

The experimental results are shown in Table 3 and FIG. 2.

Table 3: FACS assay of binding activity of 8C5.1H3L3 and AsAb-8C4703 to CLDN18.2 on surface of CHO-K1-CLDN18.2 cells

| / | 8C5.1H3L 3 | AsAb-8C4703 |
|---|---|---|
| $EC_{50}$ (nM) | 4.439 | 10.59 |

The results showed that under the same experimental conditions, the $EC_{50}$ of 8C5.1H3L3 and AsAb-8C4703 for binding to CHO-K1-CLDN18.2 cells was 4.439 nM and 10.59 nM, respectively. The results show that under the same experimental conditions, 8C5.1H3L3 and AsAb-8C4703 exhibit comparable binding activity to CHO-K1-CLDN18.2 cells, suggesting that 8C5.1H3L3 and AsAb-8C4703 have the activity of effectively binding to CLDN18.2 on the cell membrane surface of CHO-K1-CLDN18.2.

3. FACS assay of binding activity of anti-CLDN18.2/anti-CD47 bispecific antibody to red blood cells

Fresh blood (voluntary donations from healthy volunteers after informed consent) was isolated to obtain red blood cells, the red blood cells were washed twice with an appropriate amount of PBS, and then the density of the cells was adjusted. The cell suspension was added to a V-bottomed 96-well plate at $1 \times 10^6$ cells per sample, and an appropriate amount of 1% PBSA (PBS + 1% BSA) was added to each well. The mixture was centrifuged at $750\times$ g for 5 min, and the supernatant was discarded. The antibodies were diluted to concentrations of 900 nM, 300 nM, 100 nM, 33.33 nM, 11.11 nM, 3.70 nM, 1.23 nM, 0.41 nM, 0.041 nM, and 0.0041 nM with 1% PBSA, and the diluted antibodies were added to the corresponding samples at 100 $\mu$L/well. The cell pellet was resuspended and incubated on ice for 40 min. 150 $\mu$L of 1% PBSA was added to each well, and the mixture was centrifuged at $750\times$ g for 5 min. The supernatant was discarded, and the plate was further washed three times at 200 $\mu$L/time. A PE anti-human IgG Fc antibody (Biolegend, Cat. 409304) was diluted with 1% PBSA, and the diluted antibody was added to the corresponding sample at 100 $\mu$L/well. The cell pellet was resuspended and incubated on ice in the dark for 30 min. 150 $\mu$L of 1% PBSA was added to each well, and the mixture was centrifuged at $350\times$ g for 5 min. The supernatant was discarded, and the plate was further washed twice at 200 $\mu$L/time. 200 $\mu$L of 1% PBSA was added to each well, and the cell pellet was resuspended and then transferred to a loading tube for testing.

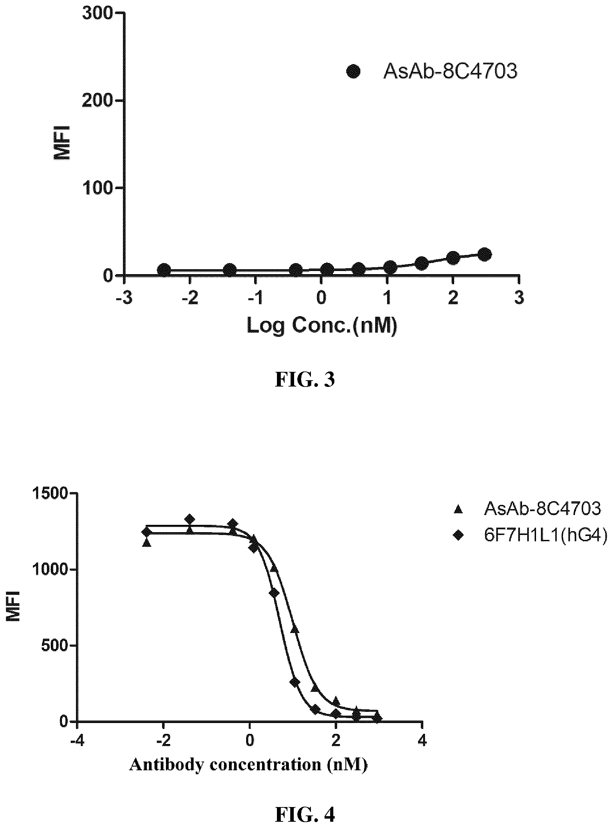

The experimental results are shown in Table 4 and FIG. 3.

Table 4: FACS assay of binding activity of anti-CLDN18.2/anti-CD47 bispecific antibody AsAb-8C4703 to red blood cells

|  | AsAb-8C4703 |
| --- | --- |
| $EC_{50}$ (nM) | 50.68 |
| MFImax | 24.32 |

The results showed that the $EC_{50}$ of the anti-CLDN18.2/anti-CD47 bispecific antibody AsAb-8C4703 for binding to red blood cells was 50.68 nM.

The results show that the anti-CLDN18.2/anti-CD47 bispecific antibody AsAb-8C4703 only has very weak binding activity to red blood cells.

Example 2: Competitive Binding of Anti-CLDN18.2/Anti-CD47 Bispecific Antibody to Cell Membrane Surface Antigen CD47

The anti-CLDN18.2/anti-CD47 bispecific antibody in competing with hSIRP$\alpha$V2 for binding to cell membrane surface antigen CD47 was assayed by competitive flow cytometry. The procedures were as follows:
CHO-K1-CLDN18.2-CD47 cells were digested conventionally and transferred to a V-bottomed 96-well plate at $3 \times 10^5$ cells/well. 100 $\mu$L of 1% PBSA was then added to each well, and the mixture was centrifuged and washed. 50 $\mu$L of hSIRP$\alpha$V2-hFc-mFe (produced by Akeso Biopharma Inc., Batch No.: 20210120) was added to each tube, and the mixture was well mixed to a final concentration of 3 nM and incubated on ice for 30 min. Corresponding serially diluted antibodies (at final concentrations of 900 nM, 300 nM, 100 nM, 33.3 nM, 11.1 nM, 3.7 nM, 1.23 nM, 0.41 nM, 0.041 nM, and 0.0041 nM, respectively) were added at 50 $\mu$L per sample, and the mixture was incubated on ice for 30 min. 150 $\mu$L of 1% PBSA was added to each well, and the mixture was centrifuged at $1000\times$ g for 5 min. The supernatant was discarded, and the plate was further washed three times at 200 $\mu$L/time. APC Streptavidin (Biolegend, Cat. 405207) was diluted with 1% PBSA (300-fold diluted), and the diluted antibody was added to corresponding samples at 100 $\mu$L/well. The cell pellet was resuspended and incubated on ice in the dark for 30 min. 150 $\mu$L of 1% PBSA was added to each well, and the mixture was centrifuged at $1000\times$ g for 5 min. The supernatant was discarded, and the plate was further washed twice at 200 $\mu$L/time. 200 $\mu$L of 1% PBSA was added to each well, and the cell pellet was resuspended and then transferred to a loading tube for testing.

The results are shown in FIG. 4, and the $EC_{50}$ values of the samples are shown in Table 5. By fluorescence quantitative analysis and curve fitting, the competitive binding $EC_{50}$ values of the antibodies 6F7H1L1(hG4) and AsAb-8C4703 were calculated to be 5.003 nM and 10.05 nM, respectively.

Table 5: Fluorescence intensity analysis of FACS assay of 6F7H1L1(hG4) in competing with AsAb-8C4703 for binding to CHO-K1-CLDN18.2-CD47 surface antigens

|  | 6F7H1L1(hG4 ) | AsAb-8C4703 |
| --- | --- | --- |
| $EC_{50}$ (nM) | 5.003 | 10.05 |

The results show that the antibodies 6F7H1L1(hG4) and AsAb-8C4703 can effectively block the binding of hSIRPαV2 to CD47 on the surface of CHO-K1-CLDN18.2-CD47 host cells in a dose-dependent manner.

Example 3: Effect of Anti-CLDN18.2/Anti-CD47 Bispecific Antibody on Agglutination of Normal Human Red Blood Cells (RBCs)

A Dextran T500 solution at 0.2 mg/mL was prepared. Fresh blood (voluntary donations from healthy volunteers after informed consent) was isolated to obtain red blood cells, the red blood cells were washed twice with an appropriate amount of PBS, and then the density of the cells was adjusted. The cells were seeded into a 96-well plate at 50 μL/well (with a cell number of $1 \times 10^7$/well), and 50 μL of corresponding antibodies (with antibody working concentrations of 3000 nM, 1000 nM, 333.3 nM, 111.1 nM, 37.0 nM, 12.3 nM, 4.1 nM, 1.4 nM, 0.14 nM, and 0.014 nM) were added to each well. 50 μL of Dextran T500 (0.1 mg/mL) was added in the positive control group, 50 μL of PBS was added in the negative control group, and hIgG1 antibody (produced by Akeso Biopharma Inc., Batch No.: 20190410) and hIgG4 antibody (produced by Akeso Biopharma Inc., Batch No.: 20190910) were added in the isotype control group. The system was incubated at 37 °C for 2-4 h, and the agglutination of red blood cells was examined and photographed.

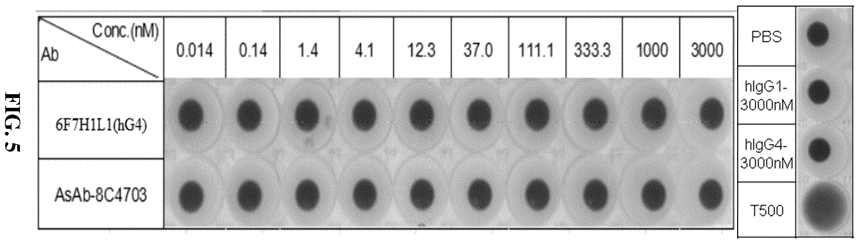

The agglutination of normal human red blood cells induced by 6F7H1L1(hG4) and AsAb-8C4703 is shown in FIG. 5.

The results showed that 6F7H1L1(hG4) and AsAb-8C4703 exhibited no effect on the agglutination of red blood cells at a concentration of 3000 nM or lower, indicating good safety.

Example 4: Phagocytosis of Tumor Cells by Macrophages Promoted by Anti-CLDN18.2/Anti-CD47 Bispecific Antibody

In this example, HPMMs were used as effector cells, KATOIII-CLDN18.2 was used as target cells, and antibody-dependent cellular phagocytosis (ADCP) activity mediated by antibodies 8C5.1H3L3, 6F7H1L1(hG4), and AsAb-8C4703 was assayed. The procedures were as follows:
KATOIII-CLDN18.2 (KATOIII cells overexpress CLDN18.2, and are obtained from Cell Resource Center, Shanghai Institutes for Biological Sciences, Chinese Academy of Sciences) was collected, washed once with PBS, and centrifuged at 170× g for 5 min. The cells were counted and analyzed for viability. CFSE (Biolegend, Cat. 423801) was diluted to a concentration of 2.5 μM with PBS, and an appropriate amount of the diluted CFSE was taken to resuspend the cells (staining density: 10 million cells/mL). The cells were incubated in an incubator for 20 min, and 6 mL of 1640 complete medium (containing 10% FBS) was added to stop the staining. The cells were centrifuged at 170× g for 5 min, and the supernatant was discarded. 1 mL of 1640 complete medium was added to resuspend the cells. The cells were incubated in an incubator for 10 min, and adjusted to a density of 150,000 cells/tube.

The antibodies (with working concentrations of 1 nM, 10 nM, 100 nM, and 300 nM) were diluted with a 1640 complete medium, and isotype controls hIgG1 (produced by Akeso Biopharma Inc., Batch No.: 20190410) and hIgG4 (produced by Akeso Biopharma Inc., Batch No.: 20190910) (both with a working concentration of 300 nM) were set.

HPMMs (isolated from peripheral blood of voluntary donations from healthy volunteers after informed consent) were collected, counted and analyzed for viability. The cell suspension was added to a 1.5 mL EP tube at 50000 cells/tube and centrifuged at 1200× g for 5 min, and the supernatant was discarded. 50 μL of target cell suspension and 50 μL of antibodies were placed in a 1.5 mL EP tube, and the mixture was incubated in an incubator for 2 h.

700 μL of 1% PBSA (PBS + 1% BSA) was added to each well, the mixture was centrifuged at 1200× g for 5 min, and the supernatant was discarded. An APC anti-mouse/human CD11b antibody (Biolegend, Cat. 101212) was diluted with 1% PBSA (500-fold dilution), and the diluted antibody was added to the corresponding samples at 100 μL/well. The cell pellet was resuspended and incubated on ice for 30 min. 800 μL of 1% PBSA was added to each well, and the mixture was centrifuged at 1200× g for 5 min. The supernatant was discarded, and the plate was further washed twice at 800 μL/time. 200 μL of 1% PBSA was added to each well, and the cell pellet was resuspended and then transferred to a loading tube for testing.

Macrophages in the system were APC-CD11b+ positive, and macrophages involved in phagocytosis were APC-CD11b+ and CFSE+ dual positive. The phagocytic index was determined as the ratio of the number of dual positive cells to the number of APC positive cells, and the antibody-mediated ADCP activity was evaluated. The ADCP activity of each group, represented by P%, was calculated according to the following formula:

$$P\% = \frac{Number\ of\ macrophages\ involved\ in\ phagocytosis}{Total\ number\ of\ macrophages} \times 100\%$$

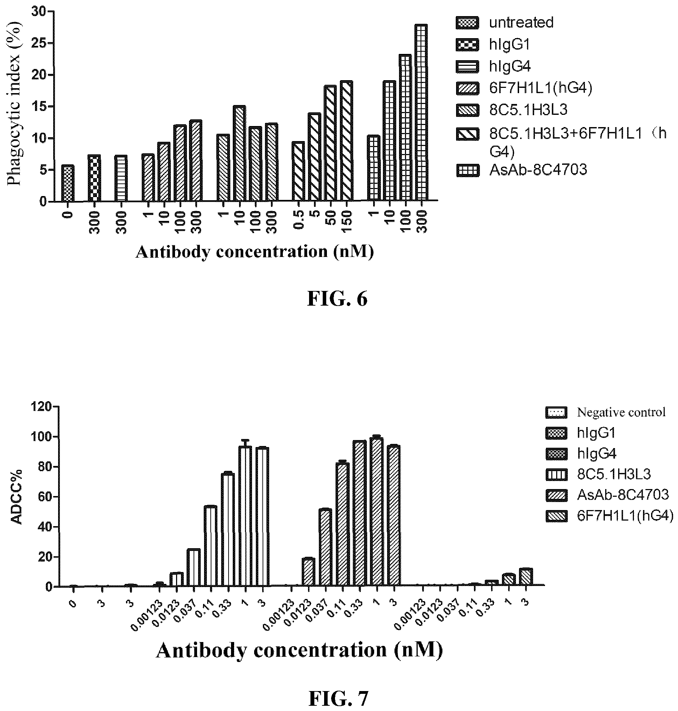

The results are shown in FIG. 6.

The results showed that the phagocytic indexes of 8C5.1H3L3, 6F7H1L1(hG4), and AsAb-8C4703 were significantly higher than those of the blank control and the isotype control, which indicates that 8C5.1H3L3, 6F7H1L1(hG4), and AsAb-8C4703 all have ADCP activity, and the ADCP activity of AsAb-8C4703 is superior to that of 8C5.1H3L3 group, 6F7H1L1(hG4) group, and 8C5.1H3L3 and 6F7H1L1(hG4) combined group.

## Example 5: ADCC Activity Assay of Anti-CLDN18.2/Anti-CD47 Bispecific Antibody

CHO-K1-CLDN18.2-CD47 cells were collected conventionally and centrifuged at $170\times$ g for 5 min, and then the cells were resuspended in a medium (RPMI-1640 + 1% FBS), and washed twice. The cells were resuspended in a medium and then counted, and the cell density was adjusted. The target cell suspension was added to a 96-well plate at 100 $\mu$L/well (about $3 \times 10^4$ target cells/well), and the antibodies 8C5.1H3L3, 6F7H1L1(hG4), and AsAb-8C4703 (all with working concentrations of 3 nM, 1 nM, 0.33 nM, 0.11 nM, 0.037 nM, 0.0123 nM, and 0.00123 nM) were diluted with a medium (RPMI-1640 + 1% FBS), and the diluted antibodies were added to corresponding wells at 50 $\mu$L/well. The system was incubated in an incubator at 37 °C for 1 h, and a negative control medium and an isotype control hIgG1 (produced by Akeso Biopharma Inc., Batch No.: 20190410) were set.

PBMC effector cells were collected conventionally and centrifuged at $170\times$ g for 5 min, and the supernatant was discarded. The cells were washed twice, resuspended in a medium (RPMI-1640 + 1% FBS), and then counted. The cell density was adjusted. 50 $\mu$L of effector cell suspension (containing about $9 \times 10^5$ effector cells/well) was added to the pre-incubated target cells, and the mixture was well mixed. The cells were incubated in an incubator with 5% $CO_2$ at 37 °C for 4 h, and centrifuged at $250\times$ g for 5 min. 100 $\mu$L of culture supernatant was carefully pipetted and transferred to a new flat-bottom 96-well plate, and 100 $\mu$L of newly prepared reaction solution was added to each well. The mixture was incubated in the dark at room temperature for 30 min, and OD values at 490 nm and 650 nm were measured. The OD value of each group = OD490nm - OD650nm.

The results are shown in FIG. 7.

The results show that compared to the isotype control, anti-CLDN18.2/anti-CD47 bispecific antibody AsAb-8C4703 has ADCC activity, is capable of specifically killing CLDN18.2 and CD47-positive cells, and has better killing activity than the corresponding monoclonal antibodies 8C5.1H3L3 and 6F7H1L1(hG4).

## Example 6: CDC Activity Assay of Anti-CLDN18.2/Anti-CD47 Bispecific Antibody

CHO-K1-CLDN18.2-CD47 cells were collected by conventional digestion, and centrifuged at $170\times$ g for 5 min. The cells were then resuspended in a medium (RPMI-1640 + 1% FBS), and washed twice. The cells were resuspended in a medium (RPMI-1640 + 1% FBS), and then counted. The cell density was adjusted. The target cell suspension was added to a 96-well plate at 100 $\mu$L/well (about $3 \times 10^4$ cells/well), and the antibodies (all with working concentrations of 100 nM, 10 nM, 1nM, and 0.1 nM) were diluted with a medium (RPMI-1640 + 1% FBS). The diluted antibodies were added to corresponding wells at 50 $\mu$L/well, and the system was pre-incubated at room temperature for 10 min. 50 $\mu$L of complement serum (at a final concentration of 2%) was added to the target cells after pre-incubation, and the mixture was well mixed. The system was incubated in an incubator with 5% $CO_2$ at 37 °C for 4 h, and centrifuged at $250\times$ g for 5 min. 100 $\mu$L of culture supernatant was carefully pipetted and transferred to a new flat-bottom 96-well plate, and 100 $\mu$L of newly prepared reaction solution was added to each well. The mixture was incubated in the dark at room temperature for 30 min, and

OD values at 490 nm and 650 nm were measured. The OD value of each group = OD490nm - OD650nm.

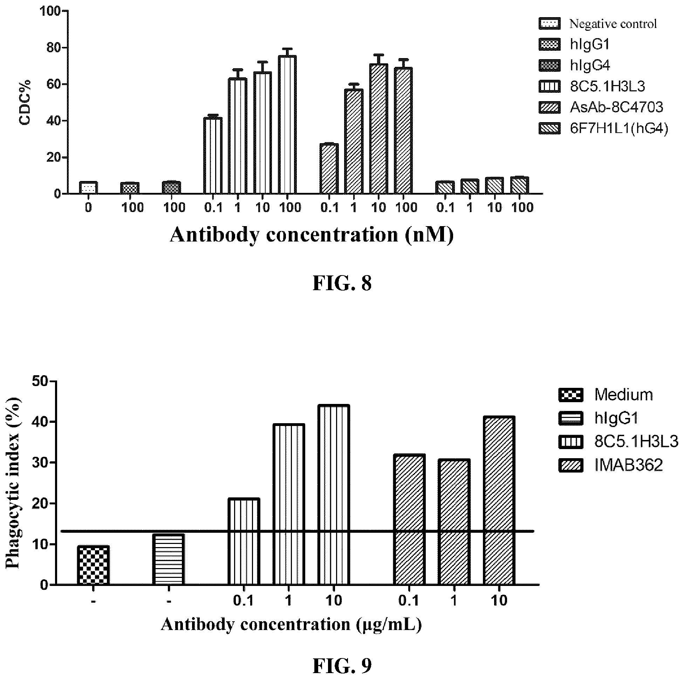

As shown in FIG. 8, compared to the isotype control hIgG1 (produced by Akeso Biopharma Inc., Batch No.: 20190410), anti-CLDN18.2/anti-CD47 bispecific antibody AsAb-8C4703 has CDC activity, can kill tumor cells by complement mediation, and has comparable activity compared to that of 8C5.1H3L3.

## Example 7: Phagocytosis of Tumor Cells by Macrophages Promoted by Anti-CLDN18.2 Antibody

In this example, the antibody-dependent cellular phagocytosis (ADCP) activity mediated by the antibody 8C5.1H3L3 was detected using MBMMs (mouse bone marrow-derived macrophages) as effector cells and CHO-K1-CLDN18.2 as target cells.

Claudiximab (IMAB362, zolbetuximab) is a human murine chimeric monoclonal antibody targeting CLDN18.2 developed by Ganymed, Germany. Claudiximab can specifically recognize and bind to the first extracellular domain (ECD1) outside the cell membrane of the CLDN18.2 protein to mediate antibody-dependent cellular phagocytosis (ADCP) etc., (Singh P, Toom S, Huang Y. Anti-claudin18.2 antibody as new targeted therapy for advanced gastric cancer [J]. Journal of Hematology & Oncology, 2017, 10(1).).

The specific method of this example is as follows:
CHO-K1-CLDN18.2 cells were collected conventionally, centrifuged at $170\times$ g for 5 min, resuspended, counted, analyzed for viability, and washed once with PBS. CFSE was diluted to 2.5 $\mu$M with PBS, and the cells were resuspended in an appropriate amount of the diluted CFSE (staining density: 10 million cells/mL) and incubated in an incubator for 20 min.

6 mL of DMEM complete medium (containing 10% FBS) was added to stop the staining. The cells were centrifuged at 170× g for 5 min, and the supernatant was discarded. 1 mL of DMEM complete medium was added and the cells were incubated in an incubator for 10 min. The antibody was diluted with a DMEM complete medium (10 μg/mL, 1 μg/mL, and 0.1 μg/mL), and an isotype control antibody and a reference antibody were designed.

Macrophages were collected and centrifuged at 170× g for 5 min. The supernatant was discarded, and the cells were counted. The cells were transferred to a 1.5 mL EP tube and centrifuged at 1200× g for 5 min, and the supernatant was discarded. A suspension of tumor cell and antibody incubated for 30 min was added to a 1.5 mL EP tube containing macrophages, and the cells were resuspended. The mixture was well mixed, and incubated in an incubator at 37 °C for 2 h. 800 μL of normal-temperature 1% PBSA was added to each tube, and the mixture was centrifuged at 1200× g for 5 min. The supernatant was discarded, and the cells were washed once with 800 μL of PBSA. A 600-fold diluted APC anti-mouse/human CD11b antibody (Biolegend, Cat. No.: 101212) was added to corresponding samples at 100 μL/sample, and the mixture was well mixed and incubated on ice for 40 min. 800 μL of 1% PBSA was added to each tube, and the mixture was centrifuged at 1200× g for 5 min. The supernatant was discarded, and each tube was washed once with 200 μL of PBSA. 200 μL of 1% PBSA was added to each tube for resuspending. The mixture was then transferred to a flow cytometry tube for testing.

The results are shown in FIG. 9.

The results show that the phagocytic indexes of 8C5.1H3L3 and IMAB362 (reference antibody) (produced by Akeso Biopharma Inc., Batch No.: 20190704) were significantly higher than that of the blank control and the isotype control, indicating that 8C5.1H3L3 and the reference antibody IMAB362 both have ADCP activity and that 8C5.1H3L3 has better ADCP activity than IMAB362.

## Example 8: Assay of Binding Activity of Anti-CLDN18.2/Anti-CD47 Bispecific Antibody to Antigens by ELISA

1. The binding activity of AsAb-8C4703, 8C5.1H3L3, and IMAB362 to the antigen human CLDN18.2 was assayed by indirect ELISA, separately. The procedures were as follows:

An ELISA plate was coated with human CLDN18.2 at 2 μg/mL and incubated at 4 °C overnight. Then the ELISA plate coated with the antigen was washed once with PBST and then blocked with a PBST solution containing 1% BSA as a blocking solution at 37 °C for 2 h. After blocking, the ELISA plate was washed 3 times with PBST. The antibodies serially diluted with PBST solution (the dilution gradients for the antibody are shown in Table 6) were added. The ELISA plate containing the test antibodies was incubated at 37 °C for 30 min and then washed 3 times with PBST. After washing, a working solution of an HRP-labeled goat anti-human IgG FC (H+L) (Jackson, Cat. No.: 109-035-098) secondary antibody diluted at a ratio of 1:5000 was added, and then the plate was incubated at 37 °C for 30 min. After the incubation was completed, the plate was washed 3 times with PBST, TMB (Neogen, 308177) was added for chromogenesis in the dark for 5 min, and then a stop solution was added to terminate the chromogenic reaction. The ELISA plate was put into a microplate reader immediately, and the OD value of each well in the ELISA plate was read at 450 nm. The data were analyzed and processed by SoftMax Pro 6.2.1.

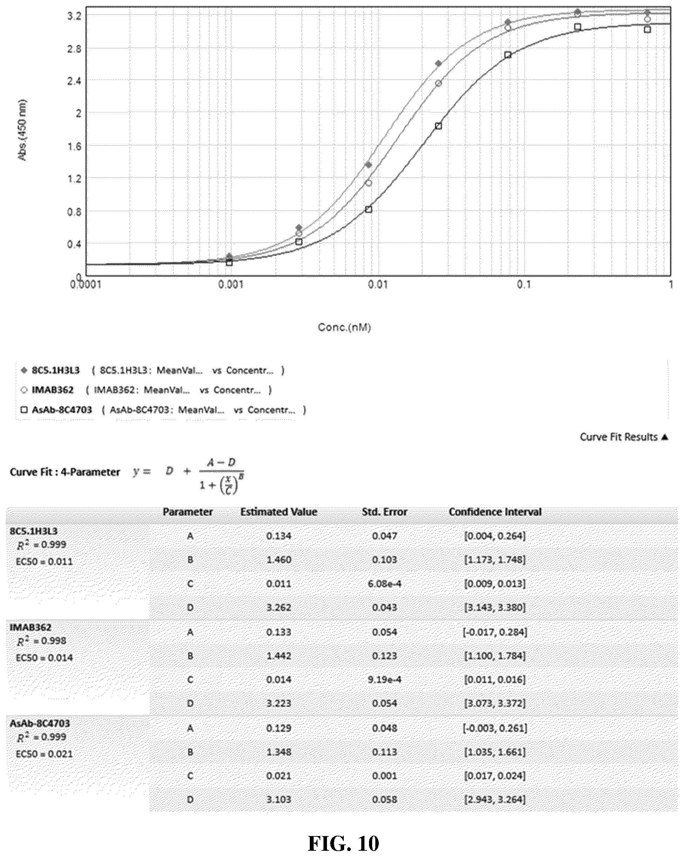

The assay results are shown in Table 6 and FIG. 10.

Table 6: Assay of binding of AsAb-8C4703, 8C5.1H3L3, and IMAB362 to human CLDN18.2

| Antibody concentration (nM) | Human CLDN18.2 (2 μg/mL) | | | | | |
| --- | --- | --- | --- | --- | --- | --- |
| | 8C5.1H3L3 | IMAB362 | | AsAb-8C4703 | | |
| 0.700 | 3.211 | 3.229 | 3.153 | 3.143 | 3.050 | 2.986 |
| 0.233 | 3.241 | 3.227 | 3.211 | 3.183 | 3.090 | 3.007 |
| 0.078 | 3.112 | 3.096 | 3.050 | 3.035 | 2.842 | 2.562 |
| 0.026 | 2.599 | 2.600 | 2.374 | 2.322 | 1.934 | 1.726 |
| 0.009 | 1.351 | 1.357 | 1.142 | 1.133 | 0.856 | 0.762 |
| 0.003 | 0.601 | 0.573 | 0.513 | 0.524 | 0.432 | 0.386 |
| 0.001 | 0.235 | 0.232 | 0.204 | 0.195 | 0.160 | 0.163 |
| 0 | 0.088 | 0.088 | 0.082 | 0.084 | 0.084 | 0.091 |

(continued)

| Secondary antibody | Goat Anti Human IgG Fc, HRP conjugated (1: 5000) | | |
|---|---|---|---|
| EC$_{50}$ (nM) | 0.011 | 0.014 | 0.021 |

FIG. 10 shows that AsAb-8C4703, 8C5.1H3L3, and IMAB362 can effectively bind to human CLDN18.2 in a dose-dependent manner. By quantitative analysis of the absorbance of the bound antibodies, the binding efficiency EC$_{50}$ values of the antibodies AsAb-8C4703, 8C5.1H3L3, and IMAB362 (as a control) obtained by curve fitting calculation were 0.021 nM, 0.011 nM, and 0.014 nM, respectively.

The results show that under the same experimental conditions, AsAb-8C4703 and 8C5.1H3L3 exhibit effective activity for binding to human CLDN18.2.

2. The binding activity of AsAb-8C4703 and 6F7H1L1(hG4) to the antigen CD47-Igv-TEV-his was assayed by indirect ELISA, separately. The procedures were as follows:

An ELISA plate was coated with CD47-Igv-TEV-his at 2 μg/mL and incubated at 4 °C overnight. Then the ELISA plate coated with the antigen was washed once with PBST and then blocked with a PBST solution containing 1% BSA as a blocking solution at 37 °C for 2 h. After blocking, the ELISA plate was washed 3 times with PBST. The antibodies serially diluted with PBST solution (the dilution gradients for the antibody are shown in Table 7) were added. The ELISA plate containing the test antibodies was incubated at 37 °C for 30 min and then washed 3 times with PBST. After washing, a working solution of an HRP-labeled goat anti-human IgG FC (H+L) (Jackson, Cat. No.: 109-035-098) secondary antibody diluted at a ratio of 1:5000 was added, and then the plate was incubated at 37 °C for 30 min. After the incubation was completed, the plate was washed 3 times with PBST, TMB (Neogen, 308177) was added for chromogenesis in the dark for 5 min, and then a stop solution was added to terminate the chromogenic reaction. The ELISA plate was put into a microplate reader immediately, and the OD value of each well in the ELISA plate was read at 450 nm. The data were analyzed and processed by SoftMax Pro 6.2.1.

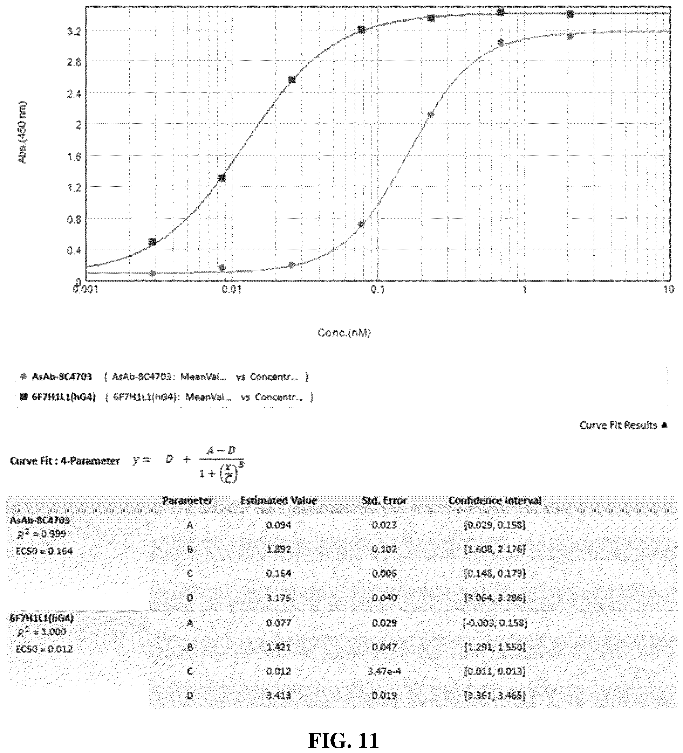

The assay results are shown in Table 7 and FIG. 11.

Table 7: Assay of binding of AsAb-8C4703 and 6F7H1L1(hG4) to CD47-Igv-TEV-his by ELISA

| Antibody concentration | CD47-Igv-TEV-his (2μg/mL) | | | |
|---|---|---|---|---|
| (nM) | AsAb-8C4703 | | 6F7H1L1(hG4) | |
| 2.100 | 3.096 | 3.134 | 3.387 | 3.398 |
| 0.700 | 3.026 | 3.050 | 3.415 | 3.429 |
| 0.233 | 1.987 | 2.241 | 3.331 | 3.365 |
| 0.078 | 0.676 | 0.741 | 3.192 | 3.197 |
| 0.026 | 0.182 | 0.197 | 2.518 | 2.603 |
| 0.009 | 0.157 | 0.139 | 1.266 | 1.327 |
| 0.003 | 0.072 | 0.078 | 0.477 | 0.496 |
| 0 | 0.063 | 0.064 | 0.061 | 0.056 |
| Secondary antibody | Goat Anti Human IgG Fc, HRP conjugated (1: 5000) | | | |
| EC$_{50}$ (nM) | 0.164 | | 0.012 | |

FIG. 11 shows that AsAb-8C4703 and 6F7H1L1(hG4) can effectively bind to CD47-Igv-TEV-his in a dose-dependent manner. By quantitative analysis of the absorbance of the bound antibodies, the binding efficiency EC$_{50}$ values of the antibodies AsAb-8C4703 and 6F7H1L1(hG4) obtained by curve fitting calculation were 0.164 nM and 0.012 nM, respectively.

The results show that under the same experimental conditions, AsAb-8C4703 and 6F7H1L1(hG4) exhibit effective activity for binding to CD47-Igv-TEV-his.

Example 9: Determination of Kinetic Parameters of Humanized Antibody Anti-CLDN18.2/Anti-CD47 Bispecific Antibody

The affinity constant of the antibody for human CLDN18.2 and CD47 ECD-His was determined using a Fortebio Octer molecular interaction instrument. The sample dilution buffer was PBS (0.02% Tween-20, 0.1% BSA, pH 7.4). The method for detecting the affinity of the antibody for human CLDN18.2 is as follows. The antibody at 20 nM was immobilized on an AHC sensor for 60 s. The sensor was equilibrated in a buffer for 60 s, and the binding of the immobilized antibody on the sensor to CLDN18.2 His at concentrations of 6.25-100 nM (two-fold dilution) was determined for 180 s. The protein was dissociated in the buffer for 360 s. The method for detecting the affinity of the antibody to human CD47 ECD-His is as follows. The antibody at 30 nM was immobilized on an AHC sensor for 120 s. The sensor was equilibrated in a buffer for 60 s, and the binding of the immobilized antibody on the sensor to the protein CD47 ECD-His at concentrations of 4.94-400 nM (three-fold dilution) was determined for 50 s. The protein was dissociated in the buffer for 120 s. The shaking speed of the sample plate was 1000 rpm, the temperature was 30 °C, and the frequency was 5.0 Hz. The data were analyzed by 1:1 model fitting to obtain affinity constants. The data acquisition software was Fortebio Data Acquisition 12.0, and the data analysis software was Fortebio Data Analysis HT 12.0.

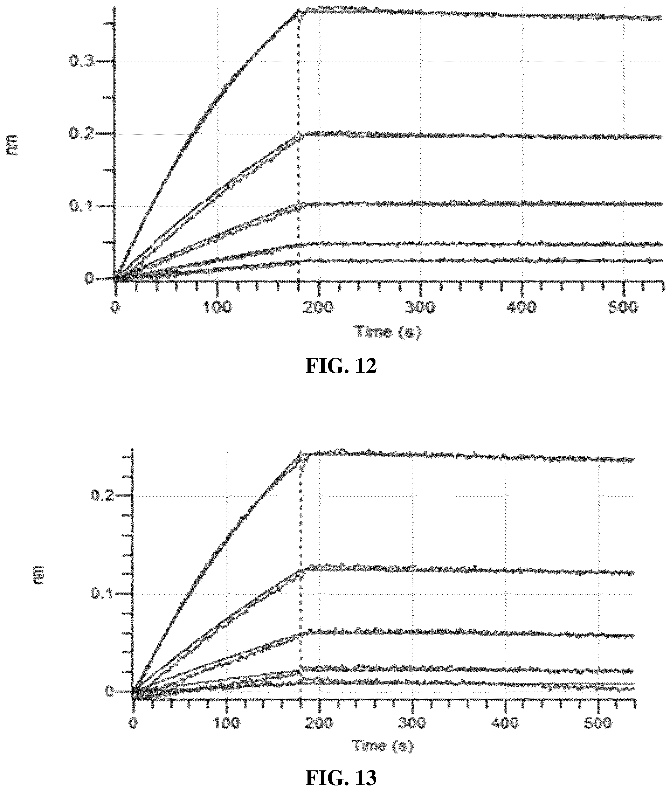

The kinetic parameters of the antibodies 8C5.1H3L3 and AsAb-8C4703 for binding to CLDN18.2 His are shown in Table 8, and the detection results of the kinetic characteristic parameters are shown in FIG. 12 and FIG. 13, respectively.

Table 8: Kinetic parameters of humanized antibodies 8C5.1H3L3 and AsAb-8C4703 for binding to CLDN18.2 His

| Name of antibody | $K_D$ (M) | kon (1/Ms) | kon error | kdis (1/s) | kdis error | Rmax (nM) |
|---|---|---|---|---|---|---|
| 8C5.1H3L3 | 1.06E-09 | 4.98E+04 | 2.34E+02 | 5.27E-05 | 1.67E-06 | 0.46-0.62 |
| AsAb-8C4703 | 1.50E-09 | 3.46E+04 | 3.33E+02 | 5.18E-05 | 2.48E-06 | 0.21-0.53 |

$K_D$ is affinity constant; kon is binding rate of antigen and antibody; kdis is dissociation rate of antigen and antibody; $K_D$ = kdis/kon.

The results show that the antibodies 8C5.1H3L3 and AsAb-8C4703 both have good affinity for the antigen CLDN18.2 His.

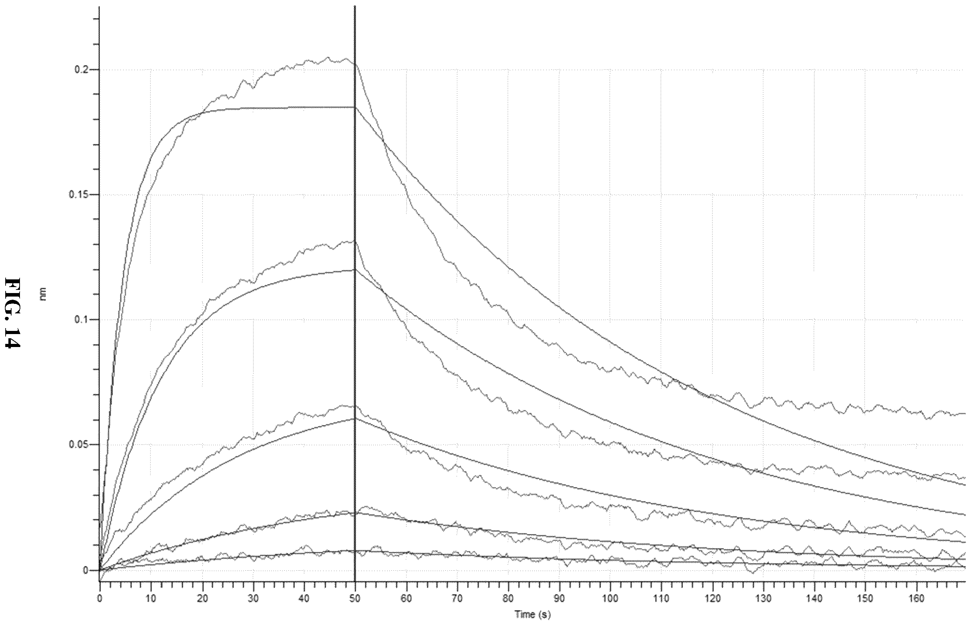

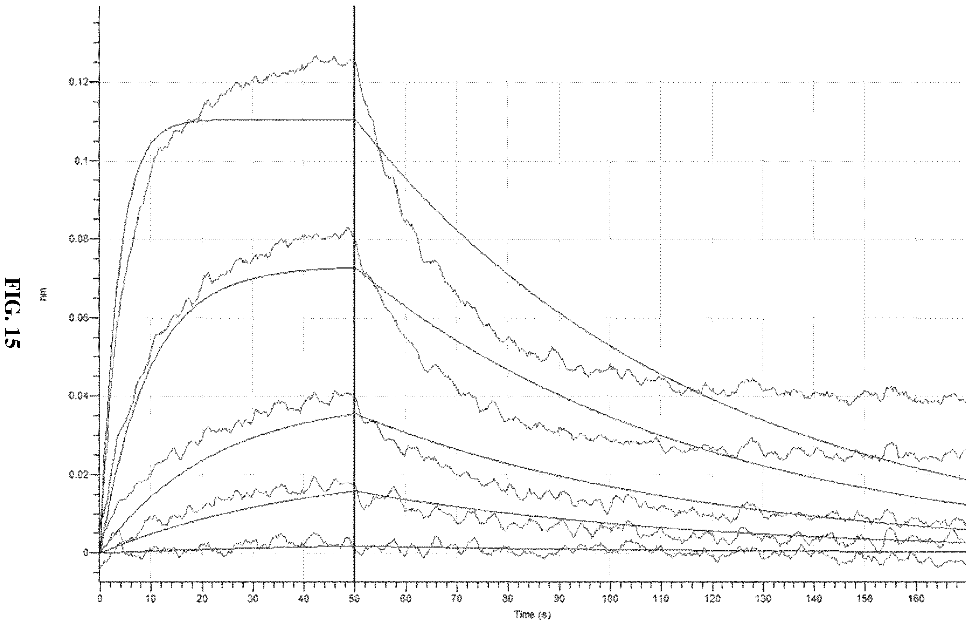

The kinetic parameters of the antibodies 6F7H1L1(hG4) and AsAb-8C4703 for binding to CD47 ECD-His are shown in Table 9, and the detection results of the kinetic characteristic parameters are shown in FIG. 14 and FIG. 15, respectively.

Table 9: Kinetic parameters of humanized antibodies 6F7H1L1(hG4) and AsAb-8C4703 for binding to CD47 ECD-His

| Name of antibody | $K_D$ (M) | kon (1/Ms) | kon error | kdis (1/s) | kdis error | Rmax (nM) |
|---|---|---|---|---|---|---|
| 6F7H1L1(hG4) | 2.73E-08 | 5.19E+05 | 9.12E+03 | 1.42E-02 | 7.83E-05 | 0.09-0.20 |
| AsAb-8C4703 | 2.17E-08 | 6.82E+05 | 1.72E+04 | 1.48E-02 | 1.12E-04 | 0.02-0.12 |

$K_D$ is affinity constant; kon is binding rate of antigen and antibody; kdis is dissociation rate of antigen and antibody; $K_D$ = kdis/kon.

The results show that the antibodies 6F7H1L1(hG4) and AsAb-8C4703 both have good affinity for the antigen.

### Example 10: Binding of Anti-CLDN18.2/Anti-CD47 Bispecific Antibody to Human Immune Cells

PBMC cells were collected conventionally, washed twice with an appropriate amount of PBS, counted, and analyzed for viability. The PBMC suspension was added to a 96-well plate at $3 \times 10^5$ cells/sample and centrifuged at $750\times$ g for 5 min, and the supernatant was discarded. A mouse IgG isotype control (Thermofisher, Cat. 10400C) at a final concentration of 5 $\mu$g/mL was added, and the mixture was blocked on ice for 20 min and centrifuged at $750\times$ g for 5 min. The supernatant was discarded. 100 $\mu$L of serially diluted antibody (with working concentrations of 900 nM, 300 nM, 100 nM, 33.3 nM, 11.1 nM, 3.7 nM, 0.37 nM, 0.037 nM, and 0.0037 nM) was added, and isotype controls hIgG1 (produced by Akeso Biopharma Inc., Batch No.: 20170424) and hIgG4 (produced by Akeso Biopharma Inc., Batch No. 20190910) (both with a working concentration of 900 nM) were designed. The cell pellet was resuspended and incubated on ice for 40 min, and 1% PBSA (PBS + 1% BSA) was added. The mixture was centrifuged at $750\times$ g for 5 min, and the supernatant was discarded. The plate was further washed 3 times. 100 $\mu$L of suspension of FITC anti-human CD3 (Biolegend, Cat. 344804), Brilliant Violet 421™ anti-human CD19 antibody (Biolegend, Cat. 302234), and mouse anti-human IgG Fc-AF647 (Southern Biotech, Cat. 9040-31) was added, and the cell pellet was resuspended and incubated on ice in the dark for 30 min. 1% PBSA was added to each well, and the mixture was centrifuged at $750\times$ g for 5 min. The supernatant was discarded, and the plate was further washed twice. 1% PBSA was added to each well, and the cell pellet was resuspended and then transferred to a loading tube for FACS Calibur assay. The reference antibody Hu5F9-G4 is a humanized IgG4 antibody drug developed by Forty Seven, which targets CD47 with high affinity.

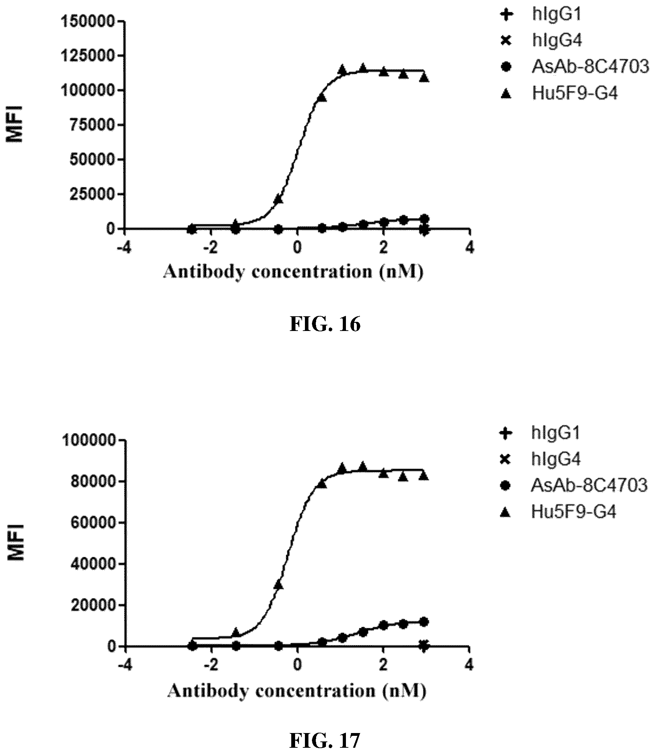

The results are shown in Table 10 and FIGs. 16 and 17.

Table 10: Binding of anti-CLDN18.2/anti-CD47 bispecific antibody to human immune cells

| / | | AsAb-8C4703 | Hu5F9-G4 |
|---|---|---|---|
| T cell | $EC_{50}$ (nM) | 37.08 | 1.076 |
| | MFImax | 6830 | 116366 |
| B cell | $EC_{50}$ (nM) | 24.14 | 0.6008 |
| | MFImax | 11871 | 88065 |

The experimental results showed that the binding MFI values of AsAb-8C4703 to CD3+ CD19- T cells and CD3- CD19+ B cells were both lower and their corresponding $EC_{50}$ values were both higher compared to those of the reference antibody Hu5F9-G4 (produced by Akeso Biopharma Inc., Batch No.: 20220303), which indicates that the binding activity of AsAb-8C4703 to both CD3+ CD19- T cells and CD3- CD19+ B cells is much weaker than that of Hu5F9-G4.

Example 11: Experiment of Inhibition of Tumor Growth *in vivo* by Anti-CLDN18.2/Anti-CD47 Bispecific Antibody

To detect the *in vivo* tumor inhibitory activity of the anti-CLDN18.2/anti-CD47 bispecific antibody, MC38-CD47-CLDN18.2 cells (MC38 mouse colon cancer cell line transfected with human CD47 and human CLDN18.2 prepared by Akeso Biopharma Inc., of which the MC38 is from Shanghai Model Organisms Center, Inc.) were first inoculated subcutaneously on the right side of 5-7 weeks old female C57BL/6 mice (purchased from GemPharmatech). The modeling and specific administration mode are shown in Table 11. After the administration, the length and width of tumors in each group were measured, and the tumor volume was calculated.

After grouping, the tumor size was measured twice weekly using a vernier caliper, and the tumor volume was calculated according to the formula $TV = 0.5 \times ab^2$, where a is the long diameter of the tumor, b is the short diameter of the tumor, and TV is the volume of the tumor. TGI (%) (tumor growth inhibition rate) was calculated from the tumor volume according to the formula: $TGI (\%) = (1 - mean_{RTV\ treat}/mean_{RTV\ vehicle}) \times 100\%$, where RTV = Vt/V0, RTV is relative tumor volume, $mean_{RTV\ treat}$ and $mean_{RTV\ vehicle}$ are mean relative tumor volume of the treatment and model groups, respectively, and Vt and V0 are tumor volume on day t and day 0, respectively. GraphPadprism software was used for plotting, and drug efficacy results were assessed according to TGI.

Table 11: Dosing regimen of anti-CLDN18.2/anti-CD47 bispecific antibody for treatment of MC38-CD47-CLDN18.2 xenograft tumor C57BL/6 mouse model (all groups from the same experiment)

| Group | Animal Number | Modeling scheme | Dosing regimen |
|---|---|---|---|
| Model group 1 | 6 | | Anti-HEL, 20mg/kg, IP, BIWx3 |
| 6F7H1L1(hG4) high-dose group | 6 | | 20mg/kg, IP, BIWx3 |
| 8C5.1H3L3 high-dose group | 6 | | 20mg/kg, IP, BIWx3 |
| AsAb-8C4703 high-dose group | 6 | | 17mg/kg, IP, BIW×3 |
| AsAb-8C4703 medium-dose group | 6 | | 5.7mg/kg, IP, BIWx3 |
| AsAb-8C4703 low-dose group | 6 | MC38-CD47-CLDN18.2 cells inoculated subcutaneously on the right side of C57BL/6 mice at $1 \times 10^6$ cells and 100 μL/mouse | 1.9mg/kg, IP, BIWx3 |
| Model group 2 | 6 | | Anti-HEL, 20mg/kg, IP, BIWx3 |
| 6F7H1L1(hG4) + 8C5.1H3L3 combination high-dose group | 6 | | 6F7H1L1(hG4), 20mg/kg+8C5.1H3L3, 20mg/kg, IP, BIWx3 |
| 6F7H1L1(hG4) + 8C5.1H3L3 combination medium-dose group | 6 | | 6F7H1L1(hG4), 6.7mg/kg+8C5.1H3L3, 6.7mg/kg, IP, BIWx3 |
| 6F7H1L1(hG4) + 8C5.1H3L3 combination low-dose group | 6 | | 6F7H1L1(hG4), 2.2mg/kg+8C5.1H3L3, 2.2mg/kg, IP, BIWx3 |
| Note: In Table 11, IP indicates intraperitoneal injection, and BIW indicates twice a week. | | | |

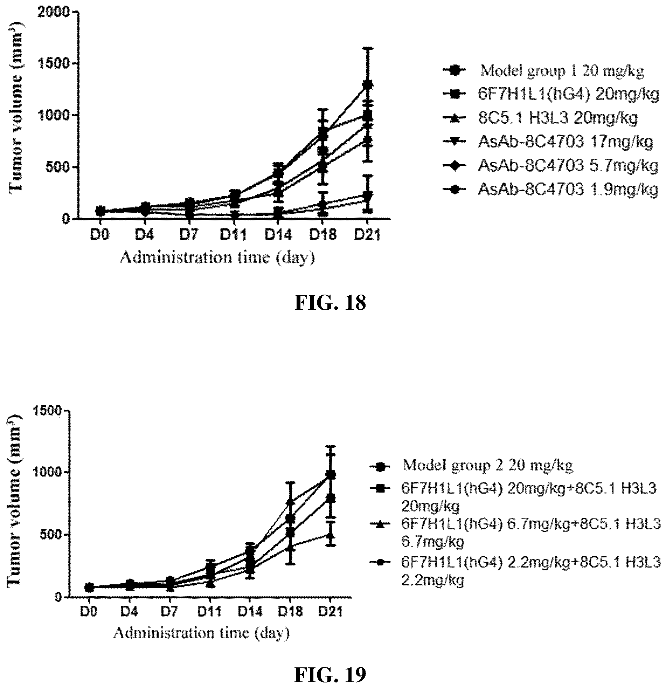

The results are shown in FIGs. 18, 19, 20, and 21.

The results showed that:

on day 21 of administration after grouping, the tumor growth inhibition TGI (%) of AsAb-8C4703 at 17 mg/kg was 85.43%, which is better than that of the 6F7H1L1(hG4) 20 mg/kg single drug group (TGI = 18.28%), the 8C5.1H3L3 20 mg/kg single drug group (TGI = 23.09%), and the 6F7H1L1(hG4) 20 mg/kg + 8C5.1H3L3 20 mg/kg combination group (TGI = 23.78%) at equimolar doses;
the tumor growth inhibition TGI (%) of AsAb-8C4703 at 5.7 mg/kg was 83.62%, which is better than that of the 6F7H1L1(hG4) 6.7 mg/kg + 8C5.1H3L3 6.7 mg/kg combination group (TGI = 51.22%) at an equimolar dose;
the tumor growth inhibition TGI (%) of AsAb-8C4703 at 1.9 mg/kg was 44.26%, which is better than that of 6F7H1L1(hG4) 2.2 mg/kg + 8C5.1H3L3 2.2 mg/kg combination group (TGI = 3.74%) at an equimolar dose.

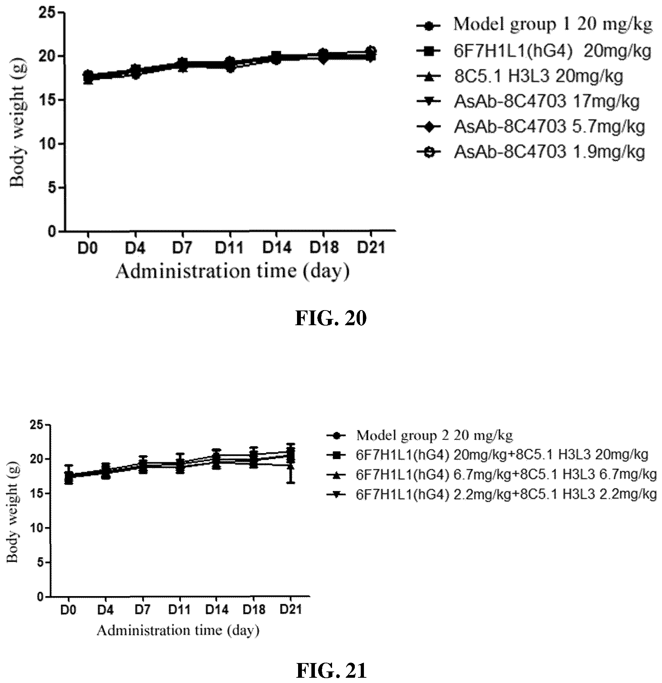

In addition, as shown in FIGs. 20 and 21, the test drugs were well tolerated by the tumor-bearing mice, and no effect on the body weight of the tumor-bearing mice was found in the groups, showing good safety.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

**Claims**

1. A bispecific antibody, comprising a first protein functional region and a second protein functional region, wherein:

the first protein functional region is an anti-CLDN18.2 antibody or an antigen-binding fragment thereof; and
the second protein functional region targets a target other than CLDN18.2 (e.g., CD47),
wherein the anti-CLDN18.2 antibody comprises a heavy chain variable region comprising HCDR1 to HCDR3 and a light chain variable region comprising LCDR1 to LCDR3, wherein:

an amino acid sequence of HCDR1 is set forth in SEQ ID NO: 31, an amino acid sequence of HCDR2 is set forth in SEQ ID NO: 32, and an amino acid sequence of HCDR3 is set forth in SEQ ID NO: 33, and
an amino acid sequence of LCDR1 is set forth in SEQ ID NO: 34, an amino acid sequence of LCDR2 is set forth in SEQ ID NO: 35, and an amino acid sequence of LCDR3 is set forth in SEQ ID NO: 36.

2. The bispecific antibody according to claim 1, wherein

an amino acid sequence of the heavy chain variable region of the anti-CLDN18.2 antibody is selected from SEQ ID NO: 28, SEQ ID NO: 37, SEQ ID NO: 39, and SEQ ID NO: 41; and
an amino acid sequence of the light chain variable region of the anti-CLDN18.2 antibody is selected from SEQ ID NO: 30, SEQ ID NO: 43, SEQ ID NO: 45, and SEQ ID NO: 47.

3. The bispecific antibody according to any one of claims 1 and 2, wherein in the anti-CLDN18.2 antibody:

the amino acid sequence of the heavy chain variable region is set forth in SEQ ID NO: 28, and the amino acid sequence of the light chain variable region is set forth in SEQ ID NO: 30;
the amino acid sequence of the heavy chain variable region is set forth in SEQ ID NO: 37, and the amino acid sequence of the light chain variable region is set forth in SEQ ID NO: 43;
the amino acid sequence of the heavy chain variable region is set forth in SEQ ID NO: 37, and the amino acid sequence of the light chain variable region is set forth in SEQ ID NO: 45;
the amino acid sequence of the heavy chain variable region is set forth in SEQ ID NO: 37, and the amino acid sequence of the light chain variable region is set forth in SEQ ID NO: 47;
the amino acid sequence of the heavy chain variable region is set forth in SEQ ID NO: 39, and the amino acid sequence of the light chain variable region is set forth in SEQ ID NO: 43;
the amino acid sequence of the heavy chain variable region is set forth in SEQ ID NO: 39, and the amino acid sequence of the light chain variable region is set forth in SEQ ID NO: 45;
the amino acid sequence of the heavy chain variable region is set forth in SEQ ID NO: 39, and the amino acid sequence of the light chain variable region is set forth in SEQ ID NO: 47;
the amino acid sequence of the heavy chain variable region is set forth in SEQ ID NO: 41, and the amino acid sequence of the light chain variable region is set forth in SEQ ID NO: 43;

the amino acid sequence of the heavy chain variable region is set forth in SEQ ID NO: 41, and the amino acid sequence of the light chain variable region is set forth in SEQ ID NO: 45;
or
the amino acid sequence of the heavy chain variable region is set forth in SEQ ID NO: 41, and the amino acid sequence of the light chain variable region is set forth in SEQ ID NO: 47.

4. The bispecific antibody according to any one of claims 1 to 3, wherein a heavy chain constant region of the anti-CLDN18.2 antibody is Ig gamma-1 chain C region or Ig gamma-4 chain C region; a light chain constant region of the anti-CLDN18.2 antibody is Ig kappa chain C region.

5. The bispecific antibody according to any one of claims 1 to 4, wherein the anti-CLDN18.2 antibody or the antigen-binding fragment thereof is selected from Fab, Fab', F(ab')$_2$, Fd, Fv, dAb, a complementarity determining region fragment, a single chain fragment variable, a humanized antibody, a chimeric antibody, or a diabody.

6. The bispecific antibody according to any one of claims 1 to 5, wherein
the anti-CLDN18.2 antibody comprises a non-CDR region derived from a non-murine species, such as from a human antibody.

7. The bispecific antibody according to any one of claims 1 to 6, wherein:

an $EC_{50}$ of the anti-CLDN18.2 antibody for binding to a cell expressing CLDN18.2 is less than or equal to 15 nM, less than or equal to 10 nM, or less than or equal to 5 nM;
and/or
an $EC_{50}$ of the anti-CLDN18.2 antibody for binding to a cell expressing both CLDN18.2 and CD47 is less than or equal to 10 nM, less than or equal to 5 nM, or less than or equal to 2 nM,
wherein preferably, the $EC_{50}$ is each determined by FACS.

8. The bispecific antibody according to any one of claims 1 to 7, wherein the anti-CLDN18.2 antibody is a monoclonal antibody produced by a hybridoma cell line LT020 deposited at China Center for Type Culture Collection (CCTCC) with a CCTCC designation of CCTCC NO. C2022124.

9. The bispecific antibody according to any one of claims 1 to 8, wherein the second protein functional region is an anti-CD47 antibody or an antigen-binding fragment thereof, wherein:
the anti-CD47 antibody comprises a heavy chain variable region comprising HCDR1 to HCDR3 and a light chain variable region comprising LCDR1 to LCDR3, wherein:

an amino acid sequence of HCDR1 is set forth in SEQ ID NO: 5, an amino acid sequence of HCDR2 is set forth in SEQ ID NO: 6, and an amino acid sequence of HCDR3 is set forth in SEQ ID NO: 7, and
an amino acid sequence of LCDR1 is set forth in SEQ ID NO: 8, an amino acid sequence of LCDR2 is set forth in SEQ ID NO: 9, and an amino acid sequence of LCDR3 is set forth in SEQ ID NO: 10.

10. The bispecific antibody according to claim 9, wherein
an amino acid sequence of the heavy chain variable region of the anti-CD47 antibody is selected from SEQ ID NO: 2, SEQ ID NO: 12, SEQ ID NO: 16, SEQ ID NO: 20, and SEQ ID NO: 24; and an amino acid sequence of the light chain variable region of the anti-CD47 antibody is selected from SEQ ID NO: 4, SEQ ID NO: 14, SEQ ID NO: 18, SEQ ID NO: 22, and SEQ ID NO: 26.

11. The bispecific antibody according to any one of claims 9 and 10, wherein in the anti-CD47 antibody:

the amino acid sequence of the heavy chain variable region is set forth in SEQ ID NO: 2, and the amino acid sequence of the light chain variable region is set forth in SEQ ID NO: 4;
the amino acid sequence of the heavy chain variable region is set forth in SEQ ID NO: 12, and the amino acid sequence of the light chain variable region is set forth in SEQ ID NO: 14;
the amino acid sequence of the heavy chain variable region is set forth in SEQ ID NO: 12, and the amino acid sequence of the light chain variable region is set forth in SEQ ID NO: 18;
the amino acid sequence of the heavy chain variable region is set forth in SEQ ID NO: 12, and the amino acid sequence of the light chain variable region is set forth in SEQ ID NO: 22;
the amino acid sequence of the heavy chain variable region is set forth in SEQ ID NO: 12, and the amino acid sequence of the light chain variable region is set forth in SEQ ID NO: 26;

the amino acid sequence of the heavy chain variable region is set forth in SEQ ID NO: 16, and the amino acid sequence of the light chain variable region is set forth in SEQ ID NO: 14;

the amino acid sequence of the heavy chain variable region is set forth in SEQ ID NO: 16, and the amino acid sequence of the light chain variable region is set forth in SEQ ID NO: 18;

the amino acid sequence of the heavy chain variable region is set forth in SEQ ID NO: 16, and the amino acid sequence of the light chain variable region is set forth in SEQ ID NO: 22;

the amino acid sequence of the heavy chain variable region is set forth in SEQ ID NO: 16, and the amino acid sequence of the light chain variable region is set forth in SEQ ID NO: 26;

the amino acid sequence of the heavy chain variable region is set forth in SEQ ID NO: 20, and the amino acid sequence of the light chain variable region is set forth in SEQ ID NO: 14;

the amino acid sequence of the heavy chain variable region is set forth in SEQ ID NO: 20, and the amino acid sequence of the light chain variable region is set forth in SEQ ID NO: 18;

the amino acid sequence of the heavy chain variable region is set forth in SEQ ID NO: 20, and the amino acid sequence of the light chain variable region is set forth in SEQ ID NO: 22;

the amino acid sequence of the heavy chain variable region is set forth in SEQ ID NO: 20, and the amino acid sequence of the light chain variable region is set forth in SEQ ID NO: 26;

the amino acid sequence of the heavy chain variable region is set forth in SEQ ID NO: 24, and the amino acid sequence of the light chain variable region is set forth in SEQ ID NO: 14;

the amino acid sequence of the heavy chain variable region is set forth in SEQ ID NO: 24, and the amino acid sequence of the light chain variable region is set forth in SEQ ID NO: 18;

the amino acid sequence of the heavy chain variable region is set forth in SEQ ID NO: 24, and the amino acid sequence of the light chain variable region is set forth in SEQ ID NO: 22;

or

the amino acid sequence of the heavy chain variable region is set forth in SEQ ID NO: 24, and the amino acid sequence of the light chain variable region is set forth in SEQ ID NO: 26.

12. The bispecific antibody according to any one of claims 9 to 11, wherein a heavy chain constant region of the anti-CD47 antibody is Ig gamma-1 chain C region or Ig gamma-4 chain C region; a light chain constant region of the anti-CD47 antibody is Ig kappa chain C region.

13. The bispecific antibody according to any one of claims 1 to 12, wherein the first protein functional region and the second protein functional region are independently a fusion protein of a single chain fragment variable or a half-molecular monovalent antibody (IgG half molecule, IgG-HM).

14. The bispecific antibody according to any one of claims 1 to 13, wherein

the first protein functional region is a fusion protein of a single chain fragment variable, and the second protein functional region is a half-molecular monovalent antibody (IgG half molecule, IgG-HM);

or

the first protein functional region is a half-molecular monovalent antibody (IgG half molecule, IgG-HM), and the second protein functional region is a fusion protein of a single chain fragment variable.

15. The bispecific antibody according to any one of claims 1 to 13, wherein

the first protein functional region is a fusion protein of a single chain fragment variable targeting CLDN18.2, and the second protein functional region is a half-molecular monovalent antibody targeting CD47;

or

the first protein functional region is a half-molecular monovalent antibody targeting CLDN18.2, and the second protein functional region is a fusion protein of a single chain fragment variable targeting CD47.

16. The bispecific antibody according to any one of claims 13 to 15, wherein the fusion protein of the single chain fragment variable consists of a single chain fragment variable, a hinge region, and an Fc fragment, or consists of a single chain fragment variable and a heavy chain constant region, wherein preferably, the hinge region and the Fc fragment are a hinge region and an Fc fragment of human IgG1; preferably, the hinge region and the Fc fragment have an amino acid sequence set forth in SEQ ID NO: 62;

the heavy chain constant region is a heavy chain constant region of human IgG1; preferably, the heavy chain constant region has an amino acid sequence set forth in SEQ ID NO: 63.

17. The bispecific antibody according to any one of claims 13 to 16, wherein

the Fc fragment in the fusion protein or the heavy chain constant region of the single chain fragment variable has a Knob mutation (e.g., S354C and T366W mutations); and
a heavy chain constant region of the half-molecular monovalent antibody is a heavy chain constant region of human IgG1, and has a Hole mutation (e.g., Y349C, T366S, L368A, and Y407V mutations).

18. The bispecific antibody according to any one of claims 1 to 17, consisting of a peptide chain set forth in SEQ ID NO: 53, a peptide chain set forth in SEQ ID NO: 56, and a peptide chain set forth in SEQ ID NO: 59,

wherein preferably, the peptide chain set forth in SEQ ID NO: 53 and the peptide chain set forth in SEQ ID NO: 56 are linked by one or more disulfide bonds of a hinge region, and the peptide chain set forth in SEQ ID NO: 52 and the peptide chain set forth in SEQ ID NO: 59 are linked by one or more disulfide bonds;
preferably, the peptide chain set forth in SEQ ID NO: 53 and the peptide chain set forth in SEQ ID NO: 56 are linked by two disulfide bonds of a hinge region, and the peptide chain set forth in SEQ ID NO: 56 and the peptide chain set forth in SEQ ID NO: 59 are linked by one disulfide bond.

19. The bispecific antibody according to any one of claims 1 to 18, wherein:

an $EC_{50}$ of the bispecific antibody for binding to a cell expressing CLDN18.2 is less than or equal to 20 nM, less than or equal to 15 nM, or less than or equal to 12 nM;
an $EC_{50}$ of the bispecific antibody for binding to CD47 on red blood cell membrane surface is greater than or equal to 20 nM, greater than or equal to 40 nM, or greater than or equal to 50 nM;
and/or
an $EC_{50}$ of the bispecific antibody for binding to a cell expressing both CLDN18.2 and CD47 is less than or equal to 10 nM, less than or equal to 5 nM, or less than or equal to 2 nM,
wherein preferably, the $EC_{50}$ is each determined by FACS.

20. The bispecific antibody according to any one of claims 1 to 19, wherein the bispecific antibody does not induce agglutination of red blood cells at a concentration of 3000 nM or less.

21. The bispecific antibody according to any one of claims 1 to 19, wherein the bispecific antibody has ADCP activity, ADCC activity, and CDC activity.

22. An isolated nucleic acid molecule encoding the bispecific antibody according to any one of claims 1 to 21.

23. A vector comprising the isolated nucleic acid molecule according to claim 22.

24. A host cell comprising the isolated nucleic acid molecule according to claim 22 or the vector according to claim 23.

25. A pharmaceutical composition comprising an effective amount of the bispecific antibody according to any one of claims 1 to 21, and one or more pharmaceutically acceptable auxiliary materials.

26. Use of the bispecific antibody according to any one of claims 1 to 21 in preparing a medicament for treating or preventing a tumor,

wherein preferably, the tumor is a CD47 and/or CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

27. The bispecific antibody according to any one of claims 1 to 21 for use in treating or preventing a tumor,

wherein preferably, the tumor is a CD47 and/or CLDN18.2-positive tumor;
preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

28. A method for treating or preventing a tumor, comprising a step of administering to a subject in need an effective amount of the bispecific antibody according to any one of claims 1 to 21, wherein wherein preferably, the tumor is a CD47 and/or CLDN18.2-positive tumor;

preferably, the tumor is one or more selected from biliary tract cancer, bronchogenic carcinoma, lymphoma, ovarian cancer, esophageal cancer, melanoma, a hematological malignancy, glioblastoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer.

29. The method for treating or preventing a tumor according to claim 28, wherein drug administration is performed before or after surgery and/or before or after radiotherapy.

30. The method for treating or preventing a tumor according to any one of claims 28 and 29, wherein

the bispecific antibody is administered at a unit dose of 0.1-100 mg per kg body weight, preferably 5-50 mg or 5-15 mg per kg body weight,
preferably, drug administration is performed once every 3 days, every 4 days, every 5 days, every 6 days, every 10 days, every 1 week, every 2 weeks, or every 3 weeks;
preferably, a route of administration is intravenous drip infusion or intravenous injection.

**FIG. 1**

**FIG. 2**

EP 4 541 821 A1

FIG. 3

FIG. 4

**FIG. 5**

**FIG. 6**

**FIG. 7**

FIG. 8

FIG. 9

♦ 8C5.1H3L3   ( 8C5.1H3L3: MeanVal... vs Concentr... )

○ IMAB362   ( IMAB362: MeanVal... vs Concentr... )

□ AsAb-8C4703   ( AsAb-8C4703: MeanVal... vs Concentr... )

Curve Fit Results ▲

Curve Fit : 4-Parameter   $y = D + \dfrac{A - D}{1 + \left(\frac{x}{C}\right)^{B}}$

| | Parameter | Estimated Value | Std. Error | Confidence Interval |
|---|---|---|---|---|
| **8C5.1H3L3**<br>$R^2 = 0.999$<br>EC50 = 0.011 | A | 0.134 | 0.047 | [0.004, 0.264] |
| | B | 1.460 | 0.103 | [1.173, 1.748] |
| | C | 0.011 | 6.08e-4 | [0.009, 0.013] |
| | D | 3.262 | 0.043 | [3.143, 3.380] |
| **IMAB362**<br>$R^2 = 0.998$<br>EC50 = 0.014 | A | 0.133 | 0.054 | [-0.017, 0.284] |
| | B | 1.442 | 0.123 | [1.100, 1.784] |
| | C | 0.014 | 9.19e-4 | [0.011, 0.016] |
| | D | 3.223 | 0.054 | [3.073, 3.372] |
| **AsAb-8C4703**<br>$R^2 = 0.999$<br>EC50 = 0.021 | A | 0.129 | 0.048 | [-0.003, 0.261] |
| | B | 1.348 | 0.113 | [1.035, 1.661] |
| | C | 0.021 | 0.001 | [0.017, 0.024] |
| | D | 3.103 | 0.058 | [2.943, 3.264] |

**FIG. 10**

**FIG. 11**

**FIG. 12**

**FIG. 13**

**FIG. 14**

**FIG. 15**

**FIG. 16**

**FIG. 17**

FIG. 18

FIG. 19

FIG. 20

FIG. 21

## INTERNATIONAL SEARCH REPORT

| | International application No. |
|---|---|
| | **PCT/CN2023/100452** |

### A. CLASSIFICATION OF SUBJECT MATTER

C07K 16/46(2006.01)i; C07K 16/28(2006.01)i; C12N 15/13(2006.01)i; A61K 39/395(2006.01)i; A61P 35/00(2006.01)i

According to International Patent Classification (IPC) or to both national classification and IPC

### B. FIELDS SEARCHED

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, 超星读秀学术, CHAOXING DUXIU SCHOLAR, 中国专利生物序列检索系统, China Patent Biological Sequence Search System, CNKI, 万方, WANFANG, ISI web of knowledge, Elsevier Science Direct, Springerlink, pubmed, genbank, ENA, EMBL, Uniport, STN, 百度, BAIDU, 百度学术, BAIDU SCHOLAR, patentics: 密蛋白18.2, CLDN18.2, Claudin18.2, CD47, 抗体, 双特异性抗体, 双抗, bispecific antibody, bsAbs, LT020, C2022124, SEQ ID NOs: 1-48

### C. DOCUMENTS CONSIDERED TO BE RELEVANT

| Category* | Citation of document, with indication, where appropriate, of the relevant passages | Relevant to claim No. |
|---|---|---|
| A | WO 2021003082 A1 (PHANES THERAPEUTICS, INC.) 07 January 2021 (2021-01-07) claims 1-19 | 1-30 |
| A | CN 111518214 A (L&L BIOPHARMA CO., LTD.) 11 August 2020 (2020-08-11) claims 1-17 | 1-30 |
| A | CN 112442123 A (AKESO BIOPHARMA, INC.) 05 March 2021 (2021-03-05) claims 1-31, and sequences 5-10 | 1-30 |
| A | US 2021230272 A1 (L&L BIOPHARMA CO., LTD.) 29 July 2021 (2021-07-29) entire document | 1-30 |
| A | CN 111867630 A (L&L BIOPHARMA CO., LTD.) 30 October 2020 (2020-10-30) entire document | 1-30 |
| A | WO 2022068810 A1 (INNOVENT BIOLOGICS (SUZHOU) CO., LTD.) 07 April 2022 (2022-04-07) entire document | 1-30 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 September 2023** | **15 September 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/100452** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
| --- |
| **PCT/CN2023/100452** |

| Box No. II | Observations where certain claims were found unsearchable (Continuation of item 2 of first sheet) |
| --- | --- |

This international search report has not been established in respect of certain claims under Article 17(2)(a) for the following reasons:

1. ☑ Claims Nos.: **28-30**
   because they relate to subject matter not required to be searched by this Authority, namely:

   Claims 28-30 comprise a method for treatment of a living human or animal body, and therefore do not comply with PCT Rule 39.1(iv). In the present report, a search is conducted on the basis of the pharmaceutical use of the antibody in claims 28-30.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

| International application No. |
| --- |
| **PCT/CN2023/100452** |

| Patent document cited in search report | | | Publication date (day/month/year) | Patent family member(s) | | | Publication date (day/month/year) |
| --- | --- | --- | --- | --- | --- | --- | --- |
| WO | 2021003082 | A1 | 07 January 2021 | None | | | |
| CN | 111518214 | A | 11 August 2020 | None | | | |
| CN | 112442123 | A | 05 March 2021 | EP | 4026847 | A1 | 13 July 2022 |
| | | | | WO | 2021043220 | A1 | 11 March 2021 |
| | | | | JP | 2022545974 | A | 01 November 2022 |
| | | | | US | 2022324971 | A1 | 13 October 2022 |
| | | | | CA | 3148956 | A1 | 11 March 2021 |
| | | | | KR | 20220053665 | A | 29 April 2022 |
| | | | | BR | 112022003868 | A2 | 31 May 2022 |
| | | | | AU | 2020342192 | A1 | 21 April 2022 |
| | | | | IL | 291049 | A | 01 May 2022 |
| US | 2021230272 | A1 | 29 July 2021 | None | | | |
| CN | 111867630 | A | 30 October 2020 | JP | 2021527441 | A | 14 October 2021 |
| | | | | US | 2021230272 | A1 | 29 July 2021 |
| | | | | WO | 2019242505 | A1 | 26 December 2019 |
| | | | | EP | 3808376 | A1 | 21 April 2021 |
| | | | | EP | 3808376 | A4 | 01 September 2021 |
| WO | 2022068810 | A1 | 07 April 2022 | TW | 202216783 | A | 01 May 2022 |
| | | | | CA | 3200865 | A1 | 07 April 2022 |
| | | | | AU | 2021352532 | A1 | 25 May 2023 |
| | | | | KR | 20230079165 | A | 05 June 2023 |
| | | | | EP | 4223778 | A1 | 09 August 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4816567 A

**Non-patent literature cited in the description**

- **MATOZAKI T** ; **MURATA Y** ; **OKAZAWA H et al.** Functions and molecular mechanisms of the CD47-SIRPα signaling pathway. *Trends in cell biology*, 2009, vol. 19 (2), 72-80
- **OLDENBORG P A** ; **ZHELEZNYAK A** ; **FANG Y F et al.** Role of CD47 as a marker of self on red blood cells. *Science*, 2000, vol. 288 (5473), 2051-2054
- **JIANG P** ; **LAGENAUR CF** ; **NARAYANAN V**. Integrin-associated Protein Is a Ligand for the P84 Neural Adhesion Molecule. *Journal of Biological Chemistry*, 1999, vol. 274, 559-62
- **JAISWAL S** ; **JAMIESON C H M** ; **PANG W W et al.** *Cell*, 2009, vol. 138 (3), 271-285
- **GUNZEL, D.** ; **YU, A. S. L.** Claudins and the Modulation of Tight Junction Permeability [J. *Physiological Reviews*, 2013, vol. 93 (2), 525-569
- **CAO CHENXIN**. Research progress of transmembrane CLDN18.2 in targeted cancer therapy [J. *International Journal of Biologicals*, 2020, vol. 01, 35-36, 37-38, 39-40
- **HASHIMOTO ITARU** ; **OSHIMA TAKASHI**. Claudins and Gastric Cancer: An Overview.[J. *Cancers (Basel)*, 2022, vol. 14
- **KABAT**. Sequences of Proteins of Immunological Interest. National Institutes of Health, 1987
- **CHOTHIA** ; **LESK**. *J. Mol. Biol.*, 1987, vol. 196, 901-917
- **CHOTHIA et al.** *Nature*, 1989, vol. 342, 878-883
- **EHRENMANN F** ; **KAAS Q** ; **LEFRANC M P.** IMGT/3Dstructure-DB and IMGT/DomainGapAlign: a database and a tool for immunoglobulins or antibodies, T cell receptors, MHC, IgSF and MhcSF [J].. *Nucleic acids research*, 2009, vol. 38 (1), D301-D307
- **KÖHLER G** ; **MILSTEIN C.** Continuous cultures of fused cells secreting antibody of predefined specificity [J. *Nature*, 1975, vol. 256 (5517), 495
- **JONES et al.** *Nature*, 1986, vol. 321, 522-525
- **REICHMANN et al.** *Nature*, 1988, vol. 332, 323-329
- **PRESTA**. *Curr. Op. Struct. Biol.*, 1992, vol. 2, 593-596
- **CLARK**. *Immunol. Today*, 2000, vol. 21, 397-402
- **HOLLIGER P. et al.** *Proc. Natl. Acad. Sci. USA*, 1993, vol. 90, 6444-6448
- **POLJAK R.J. et al.** *Structure*, 1994, vol. 2, 1121-1123
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunology, Immunotherapy*, 2001, vol. 50 (1), 51-59
- Remington's Pharmaceutical Sciences. Mack Publishing Company, 1995
- **FENG YI-FAN et al.** Construction and activity analysis of HIV specific monovalent mAb 2G12. *Chinese Journal of Viral Diseases*, May 2015, vol. 5 (3), 171-175
- **ACIERNO et al.** Affinity maturation increases the stability and plasticity of the Fv domain of anti-protein antibodies. *J Mol Biol.*, 2007, vol. 374 (1), 130-46
- **HAGE T** ; **REINEMER P** ; **SEBALD W.** Crystals of a 1:1 Complex Between Human Interleukin-4 and the Extracellular Domain of Its Receptor Alpha Chain. *Eur. J. Biochem.*, 1998, vol. 258 (2), 831-6
- Molecular Cloning: A Laboratory Manual
- **SINGH P** ; **TOOM S** ; **HUANG Y.** Anti-claudin18.2 antibody as new targeted therapy for advanced gastric cancer [J. *Journal of Hematology & Oncology*, 2017, vol. 10 (1)